# EUROPÄISCHE PATENTSCHRIFT

(11) **EP 2 535 352 B1**
(45) Veröffentlichungstag und Bekanntmachung des Hinweises auf die Patenterteilung: **12.08.2015**
(21) Anmeldenummer: 11170076.1
(22) Anmeldetag: 16.06.2011
(51) Int. Cl.: C07K 16/18, C07K 16/30, A61K 39/395, C07K 16/40, A61K 39/00

(54) **Pharmazeutische Zusammensetzung mit Antikörpern gegen Katalase und Superoxiddismutase zur Tumortherapie**
Pharmaceutical compound with antibodies against catalase and superoxide dismutase for tumour treatment
Composition pharmaceutique dotée d'anticorps contre la catalase et la superoxyde dismutase pour le traitement des tumeurs

(43) Veröffentlichungstag der Anmeldung: 19.12.2012
(73) Patentinhaber: Universitätsklinikum Freiburg, 79106 Freiburg (DE)
(72) Erfinder: Bauer, Georg, 79104 Freiburg (DE)
(74) Vertreter: Keller, Günter

(56) Entgegenhaltungen:
- EP-A1- 0 349 113
- HEINZELMANN SONJA ET AL: "Multiple protective functions of catalase against intercellular apoptosis-inducing ROS signaling of human tumor cells", BIOLOGICAL CHEMISTRY, WALTER DE GRUYTER GMBH & CO, BERLIN, DE, Bd. 391, Nr. 6, 1. Juni 2010 (2010-06-01), Seiten 675-693, XP009150728, ISSN: 1431-6730
- BECHTEL WIBKE ET AL: "Catalase protects tumor cells from apoptosis induction by intercellular ROS signaling", ANTICANCER RESEARCH GREECE,, Bd. 29, Nr. 11, 1. November 2009 (2009-11-01), Seiten 4541-4557, XP009155066,
- BECHTEL WIBKE ET AL: "Modulation of Intercellular ROS Signaling of Human Tumor Cells", ANTICANCER RESEARCH, INTERNATIONAL INSTITUTE OF ANTICANCER RESEARCH, GR, Bd. 29, Nr. 11, 1. November 2009 (2009-11-01), Seiten 4559-4570, XP009139757, ISSN: 0250-7005
- MATÉS J M ET AL: "Role of reactive oxygen species in apoptosis: implications for cancer therapy", INTERNATIONAL JOURNAL OF BIOCHEMISTRY AND CELL BIOLOGY, EXETER, GB, Bd. 32, Nr. 2, 1. Februar 2000 (2000-02-01), Seiten 157-170, XP009155061, ISSN: 1357-2725
- VAN DRIEL B E M ET AL: "Expression of CuZn- and Mn-Superoxide Dismutase in Human Colorectal Neoplasms", FREE RADICAL BIOLOGY AND MEDICINE, ELSEVIER SCIENCE, US, Bd. 23, Nr. 3, 1. Januar 1997 (1997-01-01) , Seiten 435-444, XP027378672, ISSN: 0891-5849 [gefunden am 1997-01-01]
- IWASE K ET AL: "Cu/Zn- and Mn-Superoxide Dismutase Distribution and Concentration in Adrenal Tumors", JOURNAL OF SURGICAL RESEARCH, ACADEMIC PRESS INC., SAN DIEGO, CA, US, Bd. 135, Nr. 1, 1. September 2006 (2006-09-01), Seiten 150-155, XP024952627, ISSN: 0022-4804, DOI: 10.1016/J.JSS.2006.03.027 [gefunden am 2006-09-01]
- MEENTS M: "Apoptoseinduktion mittels ROS-/RNS-Signalling bei Leukämie- und Lymphomzellen im Vergleich zu EBV-immortalisierten Lymphozyten", INAUGURAL-DISSERTATION ALBERT-LUDWIGS-UNIVERSITÄT FREIBURG IM BREISGAU,, [Online] 1. Januar 2009 (2009-01-01), Seiten 1-156, XP007919962, Gefunden im Internet: URL:http://www.freidok.uni-freiburg.de/vol ltexte/6748/>

## Beschreibung

Die vorliegende Erfindung beruht auf dem unerwarteten Befund, dass sich neben Katalase auch SOD am Schutz der Tumorzellen beteiligt, wobei sich die Hemmwirkungen der beiden protektiven Enzyme komplementär unterstützen. Durch die Applikation von monoklonalen Antikörpern oder Fab-Fragmenten gegen Katalase oder SOD wird das interzelluläre ROS-Signaling reaktiviert und die Zellen in Apoptose getrieben. Es wird gezeigt, dass bei Hemmung eines der beiden Enzyme zwangsläufig durch die dadurch induzierten biochemischen Konsequenzen im Bereich der Zellmembran auch der komplementäre Schutzpartner zwangsläufig gehemmt wird.

Unerwartet und von hohem therapeutischen Wert ist der Befund, dass Antikörper oder Fab-Fragmente gegen SOD und Katalase synergistisch kooperieren. Auf dieser Basis wird eine spezifische Form der synergistischen Antikörpertherapie offenbart.

### Hintergrund der vorliegenden Erfindung

Maligne Zellen sind durch die extrazelluläre Produktion von Superoxidanionen durch membranständige NADPH-Oxidase (NOX-1) charakterisiert. Dabei wird die Aktivität der NOX durch Onkogene wie z. B. RAS unter Einschaltung von RAC gesteuert. Die durch maligne Zellen generierten Superoxidanionen und deren Dismutationsprodukt Wasserstoffperoxid sind für die Proliferation dieser Zellen und für die Aufrechterhaltung ihres transformierten Zustandes essentiell (Heinzelmann and Bauer, Biol. Chem., Vol. 391, 2010, S. 675-693).

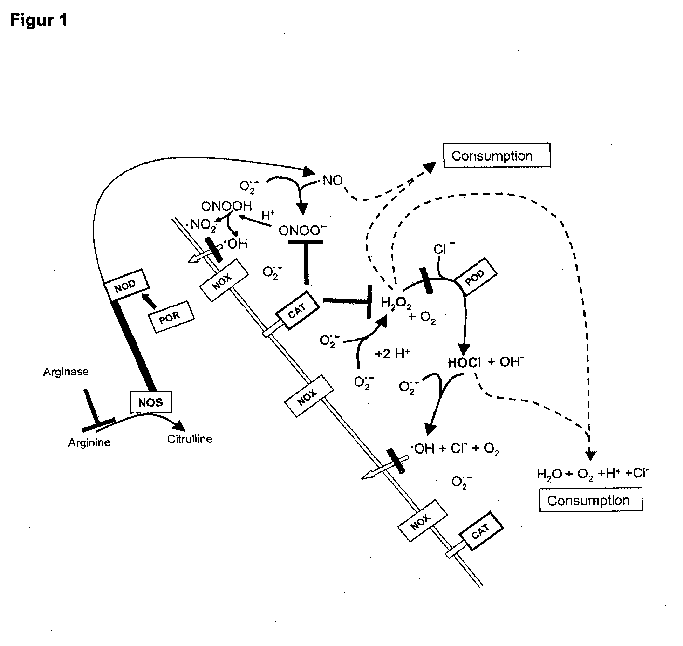

Die Kehrseite der Medaille aus der extrazellulären Produktion von Reaktiven Sauerstoffspezies (ROS) ist jedoch die Entstehung von interzellulären ROS-vermittelten Signalwegen, die sich selektiv gegen Zellen mit dem transformierten Phänotyp richten. Es sind dies die in Figur 1 gezeigten Hauptwege, nämlich der HOCI- und der NO/Peroxynitritsignalweg, sowie zwei weitere Wege von untergeordneter Bedeutung, nämlich der Nitrylchloridweg und die Metall-katalysierte Haber-Weiss-Reaktion, die in der Abbildung nicht berücksichtigt werden. Im Verlauf der Tumorprogression erwerben Tumorzellen Resistenz gegen die interzellulären ROS-Signalwege, indem sie auf ihrer Zellmembran Katalase exprimieren. Diese hemmt den HOCI- und den Nitrylchloridweg, sowie die Metall-katalysierte Haber-Weiss-Reaktion, indem sie Wasserstoffperoxid in Wasser und Sauerstoff umsetzt, und wirkt dem NO/Peroxynitritweg entgegen, indem sie Peroxynitrit zersetzt und mithilfe ihrer aktiven Zwischenstufe Compound I NO in NO₂ oxidiert und dadurch die Bildung von Peroxynitrit verhindert.

Die Aufhebung des durch Katalase vermittelten Schutzes von Tumorzellen stellt ein attraktives Konzept für die Entwicklung einer neuartigen Form der Tumortherapie dar, die sich spezifisch gegen Zellen mit dem malignen Phänotyp (aufgrund der Merkmale membranständige Katalase und Superoxidanionenproduktion) richtet und normale Zellen nicht gefährdet, da diese weder extrazelluläre Superoxidanionen produzieren noch membranständig Katalase exprimieren.

Die der vorliegenden Erfindung zugrundeliegenden Überlegungen werden in den Figuren 1 bis 6 und den nachfolgenden Ausführungen näher erläutert:

### Legende zum Schema 1 (Fig. 1)

### Tumorzellen sind durch membranständige Katalase (CAT) vor interzellulärem ROS-Signaling geschützt

Die Abbildung zeigt links den intrazellulären, rechts den interzellulären Bereich einer Tumorzelle und dazwischen das mit dem potenziellen interzellulären ROS-Signaling. In der Zellmembran befindet sich die aktivierte NADPH-Oxidase NOX-1, die extrazellulär Superoxidanionen generiert. Diese dismutieren spontan zu Wasserstoffperoxid und Sauerstoff. Die Zellen setzen Peroxidase (POD) frei, die von DUOX (nicht dargestellt) kodiert und durch Matrixmetalloproteasen abgespalten wird. Wasserstoffperoxid wird von der freien Peroxidase (POD) zu HOCI umgesetzt, welches mit Superoxidanionen zu Apoptose-induzierenden Hydroxylradikalen reagiert. Bei relativem Überschuss an Wasserstoffperoxid kommt es zur Verbrauchsreaktion von HOCI, die den HOCI-Weg schwächt. NO-Synthase (NOS) generiert NO, welches entweder von Wasserstoffperoxid in einer komplexen Konsumreaktion verbraucht wird oder mit Superoxidanionen zu Peroxynitrit reagiert. Nach Bildung von Peroxynitritsäure und deren Zerfall in Hydroxylradikale und NO₂ kommt es zur Apoptoseinduktion. Die beiden untergeordneten Signalwege Nitrylchloridweg und Metall-katalysierte Haber-Weiss-Reaktion sind in dem Schema nicht berücksichtigt. Die in dem System verfügbare Konzentration NO wird unter anderem von der Argininkonzentration, der Konzentration von Arginase und der Wirkung von NO-Dioxygenase (NOD) bestimmt. NOD ist wiederum mit Cytochrom P 450-abhängiger Oxidoreduktase (POR) assoziiert. Der NOD/POR-Komplex mindert die NO-Konzentration durch dessen Umwandlung in Nitrat.

Es ist von besonderer Bedeutung, dass die Zielzellfunktion "Superoxidanionenbildung" streng mit dem transformierten Phänotyp assoziiert und hochselektiv ist. Sie wird durch aktivierte Onkogene kontrolliert. Die Effektorfunktionen "Peroxidasefreisetzung" und "NO-Freisetzung" können jedoch sowohl von nichttransformierten als auch transformierten Zellen selbst ausgeführt werden. Daher läuft interzelluläres ROS-Signaling sowohl in der Interaktion zwischen transformierten und nichttransformierten Zellen (klassische interzelluläre Induktion der Apoptose), als auch autokrin zwischen transformierten Zellen (wie in dieser Abbildung gezeigt) ab.

Tumorzellen tragen an ihrer Aussenseite ausreichend membranständige Katalase, die Wasserstoffperoxid zerstört und dadurch den HOCI-Weg (sowie den nicht eingezeichneten Nitrylchloridweg und die Metall-katalysierte Haber-Weiss-Reaktion) hemmt. Außerdem zerstört Katalase auch Peroxynitrit und interferiert dadurch wirkungsvoll mit dem NO/Peroxynitritweg. Eine zweite negative Interaktion der Katalase mit dem NÖ/Peroxynitritweg ist aus Gründen der Übersichtlichkeit nicht eingezeichnet: Compound I der Katalase kann NO zu NO₂ oxidieren und dadurch den NO/Peroxynitritweg hemmen.

Die Hemmung der Katalase durch eine Antikörperreaktion führt zur Reaktivierung der aufgezeigten ROS-Signalwege und nachfolgend zur Apoptose.

Gegenstand dieser Patentanmeldung ist eine synergistische Wirkung unter Beteiligung von SOD, die in der folgenden Figur näher beschrieben wird.

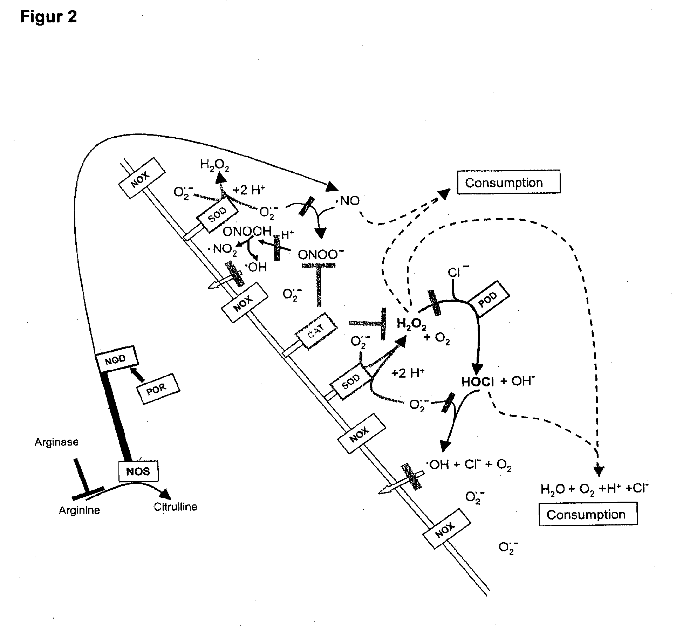

### Legende zum Schema 2 (Fig. 2)

Ohne an eine Therapie gebunden sein zu wollen, könnte das Zusammenwirken von Katalase und SOD beim Schutz von Tumorzellen vor interzellulärem ROS-Signaling auf folgenden Überlegungen beruhen:
Da auch spezifische Antikörper gegen SOD zur Wiederherstellung des ROS-Signaling führen ist der Schluss gerechtfertigt, dass sich auch SOD auf der Oberfläche der Tumorzellen befindet. Es ist erkennbar, dass SOD und Katalase sich in ihrer protektiven Wirkung ergänzen und verstärken. So verhindert SOD durch Umsetzung von freien Superoxidanionen in Wasserstoffperoxid die Bildung von Peroxynitrit aus NO und Superoxidanionen, während Katalase eventuell gebildetes Peroxynitrit abbauen kann. Dadurch wird der NO/Peroxynitritweg doppelt abgesichert. Katalase verhindert durch Abbau von Wasserstoffperoxid die Synthese von HOCI, während SOD verhindert, dass Superoxidanionen mit eventuell gebildetem HOCI interagieren und dadurch Apoptoseinduzierende Hydroxylradikale gebildet werden. Somit ist auch der HOCI-Weg durch SOD und Katalase doppelt abgesichert. Weitere protektive Mechanismen von untergeordneter Bedeutung sind aus Gründen der Übersichtlichkeit nicht aufgeführt. Es sind dies die durch Compound I der Katalase (CAT Fe^{IV} = O⁺) erfolgende Oxidation von NO zu NO₂ und die Zerstörung von Peroxynitrit durch SOD (wobei Nitroniumionen und Wasser entstehen).

Das Schema bietet allerdings keinen Erklärungsweg für die gemachte Beobachtung, dass sowohl die Hemmung der Katalase als auch Hemmung der SOD zur Apoptoseinduktion führt. Dies wird in den folgenden Schemata näher erläutert.

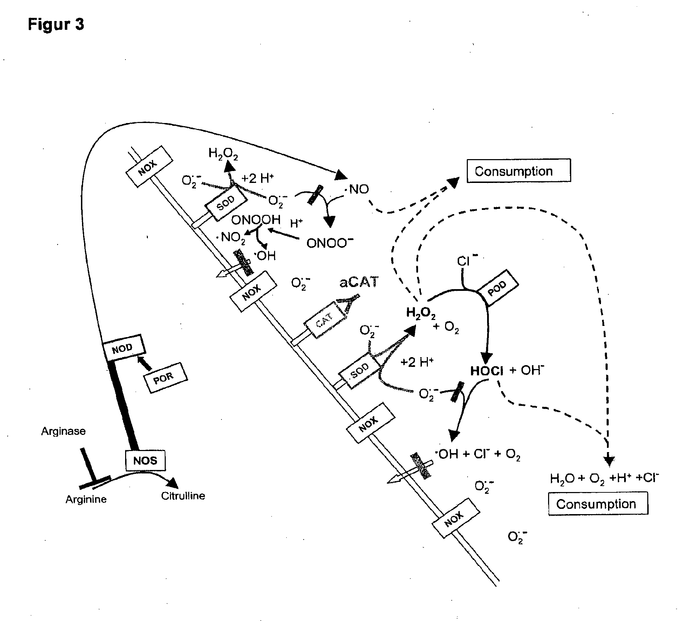

### Legende zum Schema 3 (Fig. 3)

### Hemmung der Katalase

Die Schemazeichnung macht deutlich, dass die spezifische Hemmung der Katalase durch monoklonale Antikörper (aCAT) nicht ausreichen sollte, das Signaling zu reaktivieren, da jeder der beiden Signalwege noch an zentraler Stelle durch SOD inhibiert werden sollte. Die experimentell erhobenen Befunde widersprechen jedoch dieser einfachen Ansicht.

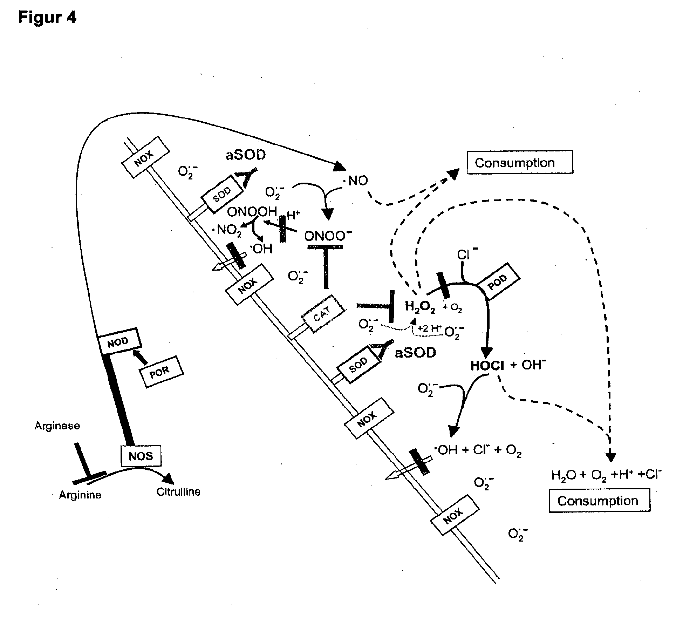

### Legende zum Schema 4 (Fig. 4)

### Hemmung der SOD

Die komplementäre Vorgehensweise, nämlich alleinige spezifische Hemmung der SOD durch Antikörper (aSOD), sollte ebenfalls zu keiner Reaktivierung des Signaling führen, da die Katalase durch Abbau von Peroxynitrit und Wasserstoffperoxid beide Signalwege blockieren kann.

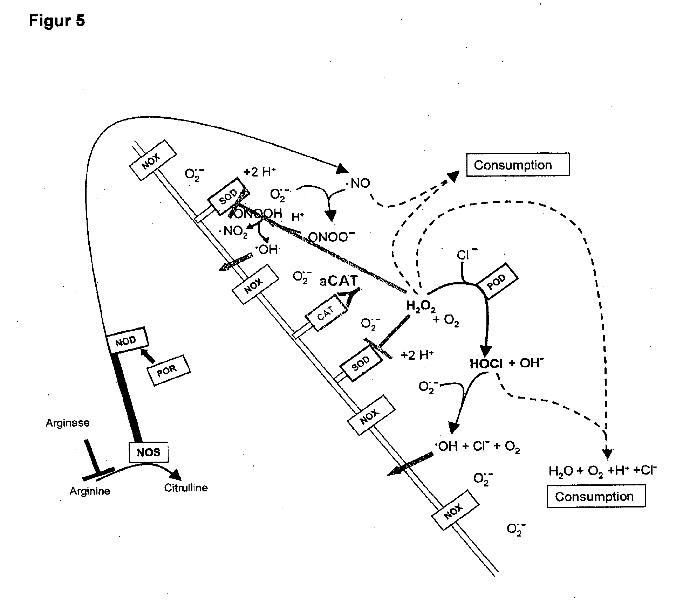

### Legende zum Schema 5 (Fig. 5)

### Hemmung der Katalase führt nachfolgend zur Hemmung der SOD

Die Schemazeichnung zeigt, wie die Hemmung der Katalase durch das nun lokal in relativ hoher Konzentration vorliegende Wasserstoffperoxid (aus spontaner und durch SOD katalysierter Dismutationsreaktion) zu einer Hemmung der SOD führt und dadurch insgesamt der Ablauf beider Signalwege möglich ist.

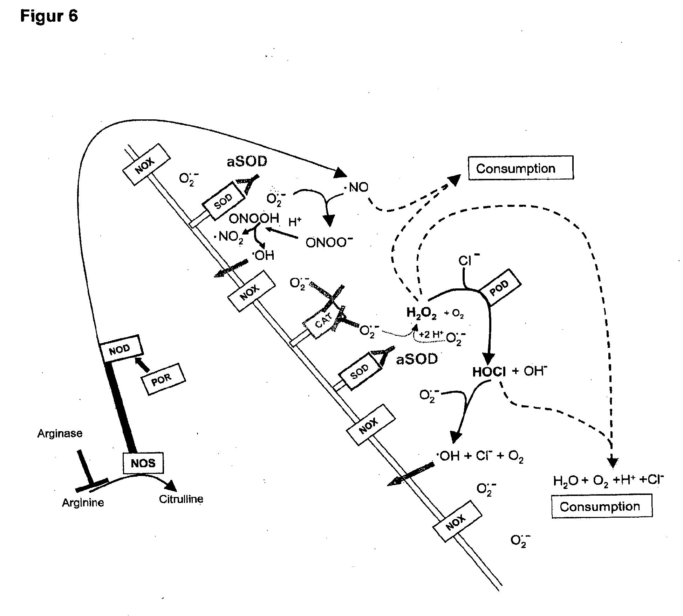

### Legende zum Schema 6 (Fig. 6)

### Hemmung der SOD führt nachfolgend zur Hemmung der Katalase

Die Schemazeichnung zeigt, dass nach Hemmung der SOD in hoher lokaler Konzentration vorliegende Superoxidanionen zu einer Hemmung der Katalase führen und dadurch die multiple Hemmung beider Signalwege aufgehoben wird.

Gegenstand der vorliegenden Patentanmeldung sind daher pharmazeutische Zusammensetzungen, die dadurch gekennzeichnet sind, dass sie wenigstens zwei unterschiedliche Antikörper oder deren biologisch aktive Fragmente enthalten, wobei der eine dieser Antikörper gegen die Katalase gerichtet ist und der andere Antikörper gegen die Superoxiddismutase gerichtet ist.

Im Rahmen der vorliegenden Erfindung werden vor allem monoklonale Antikörper eingesetzt, die bevorzugt entweder in humanisierter Form oder noch bevorzugter in vollständig humaner Form vorliegen.

Die Herstellung von monoklonalen Antikörpern ist seit den grundlegenden Arbeiten von Köhler und Milstein bekannt. Die Anwendung von monoklonalen Antikörpern, die im Rahmen der Maushybridoma-Technologie gewonnen werden, eignen sich für den pharmazeutischen Gebrauch nur sehr bedingt. Grund hierfür ist, dass bei der wiederholten Applikation am Menschen Antikörper gegen die von Mausgenen kodierten Gerüstbestandteile der Antikörper entstehen. Daher werden derartige Antikörper bevorzugt humanisiert. Hierbei werden die von Mausgenen kodierten Gerüstteile der Antikörper durch menschliche Gensequenzen ersetzt. Problematisch hierbei ist, dass die Spezifität und Affinität der Antikörper hierdurch häufig verschlechtert wird.

In einer besonders bevorzugten Ausführungsform werden die erfindungsgemäßen Antikörper mit Hilfe der sogenannten PHAGE Display-Technologie hergestellt. Hierbei entstehen Antikörper, die vollständig aus humanen Sequenzen aufgebaut sind und, die daher keine Antikörper gegen diese Antikörper bei der Applikation hervorrufen.

Bei der Herstellung der Antikörper ist wichtig, dass das Antigen, gegen das die Antikörper gerichtet sind, in einer Form präsentiert wird, die möglichst genau der Situation in vivo entspricht. Für die Herstellung der Antikörper können daher geeignete Zellen oder Zellpräparationen verwendet werden, die die räumliche Konfiguration der Antigene (hier Katalase bzw. Superoxiddismutase) in einer Form präsentieren, die der natürlichen Form möglichst genau entspricht.

Die erfindungsgemäß eingesetzten Antikörper können gegen die beiden Antigene (Katalase und Superoxiddismutase) gerichtet sein, wobei es sich um Epitope handeln kann, die auf diesen beiden Enzymen lokalisiert sind. In besonders bevorzugter Ausführungsform binden die Antikörper an die katalytischen Zentren der beiden Enzyme Katalase bzw. Superoxiddismutase). Es ist auch möglich, dass die Epitope in der Nähe der katalytischen Zentren lokalisiert sind. Aufgrund der Größe der Antikörper reicht es häufig aus, dass die Antikörper in die Nähe der katalytischen Zentren binden, weil dadurch der Zugang des Substrats an das Enzym verhindert wird.

Unter dem Begriff "Antikörper oder deren bindenden Teile" werden nicht nur komplette Antikörper der verschiedenen Antikörperklassen, insbesondere IgM, IgA und besonders bevorzugt IgG verstanden. Es kann sich vielmehr auch nur um die bindenden Fragmente, die sogenannten Fab-Fragmente handeln, die spezifisch an die Epitope binden.

Es ist in einer weiteren Ausführungsform der vorliegenden Erfindung möglich, zwei Fab-Fragmente miteinander zu sogenannten Diabodies zu koppeln, wobei das eine Fab-Fragment an die Katalase bindet und das andere Fab-Fragment an die Superoxiddismutase. Durch diese Koppelung ist es möglich, eine besonders hohe Wirksamkeit zu erzielen.

Die erfindungsgemäßen pharmazeutischen Zusammensetzungen enthaltend auf der einen Seite eine Antikörper bzw. bindende Fragmente davon, die gegen Katalase gerichtet sind, und andererseits einen Antikörper bzw. bindende Fragmente davon, die gegen die Superoxiddismutase gerichtet sind, werden bevorzugt bei der Therapie von Tumorerkrankungen eingesetzt. Besonders gute Wirksamkeit wurde bei folgenden Tumorerkrankungen beobachtet: Karzinome, insbesondere des Magens; Ewing-Sarkome, Neuroblastome, Zervixkarzinome.

Die nachfolgenden Versuche wurden mit folgenden Antikörpern bzw. Fab-Fragmenten durchgeführt:
(1) Monoklonaler Antikörper (Maus; IgG1) gegen humane Katalase (Clone CAT-505) (Chargennummer: 088K4809) Hersteller Sigma Aldrich, Schnelldorf, Deutschland.
2) Monoklonaler Antikörper (Maus, IgG1) gegen humane SOD-1 (Clone SD-G6) (Chargennummer 035K4823). Hersteller Sigma Aldrich, Schnelldorf, Deutschland.
3) Rekombinante humane Fab-Fragmente gegen humane Katalase, Format Fab-V5Sx2, hergestellt von AbDSerotec aus einer HuCAL® Library (geschützt durchEP 859841; US 6,300,064; U 725609). Eingesetzt wurden die Fragmente #AbD15562 und #AbD15563 mit Katalase-Hemmwirkung und das Fragment AbD15558 das an Katalase bindet, diese aber nicht hemmt.
4) Rekombinante humane Fab-Fragmente gegen humane SOD, Format Fab-V5Sx2, hergestellt von AbDSerotec aus einer HuCAL® Library. Eingesetzt wurden die Fragmente #AbD15660 und #AbD15662 mit SOD-Hemmwirkung und das Fragment AbD15661, das an SOD bindet, diese aber nicht hemmt. Es wurden die in der nachfolgenden Liste aufgeführten Abkürzungen verwendet:

### Liste der Abkürzungen

- AEBSF: 4-(2-Aminoethyl)-benzenesulfonyl fluoride (Hemmstoff der NADPH-Oxidase)
- ABH: 4-Aminobenzoyl hydrazid (Peroxidasehemmstoff)
- 3-AT: 3-Aminotriazol (Katalasehemmstoff)
- aCAT: Antikörper gegen Katalase
- aSOD: Antikörper gegen SOD
- 3-Br-7-NI: 3-Bromo-7-Nitroindazol (Selektiver Inhibitor für neuronale NOS (nNOS))
- CAT: Katalase
- Compound I: Aktivierte Zwischenstufe der Katalase mit der Formel CAT Fe^{IV} = O^{+.}. Compound I wird bei der Reaktion von Katalase mit einem Molekül Wasserstoffperoxid oder Peroxynitrit gebildet.
- DEA NONOate: 2-(N,N-Diethylamino)-diazenolate-2-oxide . diethylammonium salt (schnell zerfallender NO-Donor)
- Duox: Duale Oxidase (membranständiges Enzym das aus einer NADPH-Oxidase- und einer Peroxidasedomäne besteht. Die Peroxidasedomäne wird mithilfe von Proteasen abgespalten)
- EUK 134: Chloro[[2,2'-[1,2-ethanediylbis[(nitrilo-κN)methylidyne]]bis[6-meethoxyphenolato-KO]]]-manganese) (Katalasemimetikum)
- FBS: Fetales Bovines Serum
- FeTPPS: 5-, 10-, 15-, 20-Tetrakis(4-sulfonatophenyl)porphyrinato iron(III) chloride (Peroxynitrite decomposition catalyst)
- MnTE-2PyP: Mn(III) meso-tetrakis(N-ethyl-2-pyridyl)porphyrin Pentachloride (zellgängiges SOD-Mimetikum)
- L-NAME: N-ω-nitro-L-arginine methylester hydrochloride (NOS-Inhibitor)
- NO: Nitric oxide (Stickstoffmonoxid)
- NOD: Nitric oxide dioxygenase (oxidiert NO zu Nitrat)
- NOS: NO-Synthase
- NOX: NADPH-oxidase (hier insbesondere die membranständige NOX-1)
- POD: Peroxidase
(in diesem Kontext kommt insbesondere die Fähigkeit bestimmter Peroxidasen zum Tragen, die in Gegenwart von Wasserstoffperoxid Chlorid zu HOCI oxidieren können)
- PON: Peroxynitrit
- POR: Cytochrom P 450 Oxidoreduktase
- RAS, RAC: Onkogene
- ROS: Reactive oxygen and nitrogen species
(Radikalische und nichtradikalische Spezies wie Superoxidanionen, Hydroxylradikale, Stickstoffmonoxid, Wasserstoffperoxid, HOCI, Peroxynitrit, etc.)
- siRNA: small interfering RNA
(Reagenz zum spezifischen Herunterregulieren der Synthese definierter Genprodukte
- SOD: Superoxiddismutase
(hier insbesondere SOD-1 (Cu⁺⁺ im aktiven Zentrum der Tumorzellen und MnSOD aus Bakterien für analytische Zwecke)
- TGF-beta: Transforming growth factor type beta

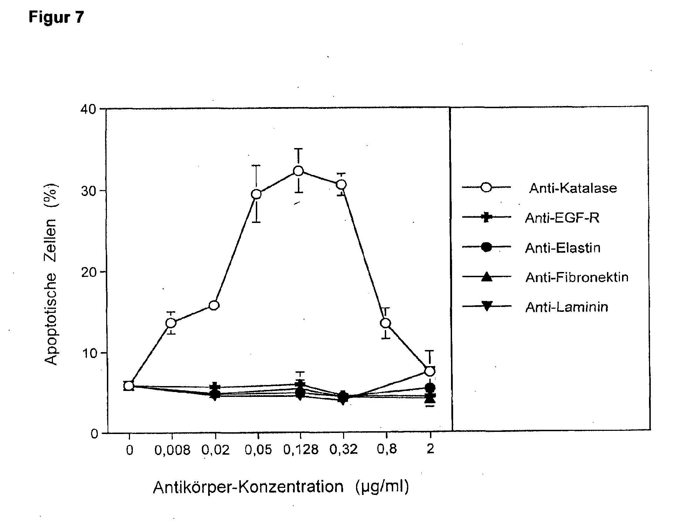

Im Rahmen der vorliegenden Erfindung wurden Versuche durchgeführt, wobei die Versuchsergebnisse in den Figuren 7 bis 20 dargestellt wurden.

### Beispiel 1: Spezifische Sensitivierung von Tumorzellen für Apoptose-auslösendes ROS-Signaling durch Antikörper gegen Katalase

Die spezifische Apoptoseinduktion in MKN-45 Magenkarzinomzellen durch monoklonale Antikörper gegen Katalase wird in Fig. 7 gezeigt.

12 500 MKN-45 Zellen in 100 µl RPMI 1640 Medium, 10 % FBS wurden mit den angebenen Konzentrationen der monoklonalen Antikörper gegen Katalase, EGF-Rezeptor, Elastin, Fibronectin oder Laminin versetzt und für 4 Stunden bei 37 °C, 5 % CO₂ weiterinkubiert. Danach erfolgte die Bestimmung des Prozentsatzes apoptotischer Zellen (Doppelansätze) nach den klassischen Apoptosekriterien Membran-Blebbing, Kernkondensation und/oder Kernfragmentation.

Die Figur 7 zeigt, dass Antikörper gegen Katalase, nicht aber gegen andere membranassoziierte Proteine gerichtete Antikörper Apoptose in den Tumorzellen auslösen, wobei sich die Wirkung in Form einer Optimumskurve darstellt, wie dies auch in der Arbeit von Heinzelmann and Bauer, 2010 für den Katalasehemmstoff 3-Aminotriazol beschrieben worden ist.

Die Zugabe von monoklonalen Antikörpern gegen humane Katalase führt in der Magenkarzinomzelllinie MKN-45 zur Induktion der Apoptose. Diese Sensitivierung zeigt die Form einer Optimumskurve in bezug auf die Konzentration der Antikörper. Die Spezifität des induzierten Prozesses wird dadurch belegt, dass monoklonale Antikörper gegen eine Reihe anderer Membranstrukturen der Tumorzellen, wie z. B. EGF-Rezeptor, Elastin, Fibronectin oder Laminin zu keiner Apoptoseinduktion führen (Fig. 7). Es genügt für die Sensitivierung also nicht, dass Antikörper an eine membranständige Strukur binden. Die spezifische Bindung an Katalase, die zu einer offensichtlichen Hemmung der Funktion der Katalase führt, scheint für die Sensitivierung unerlässlich zu sein.

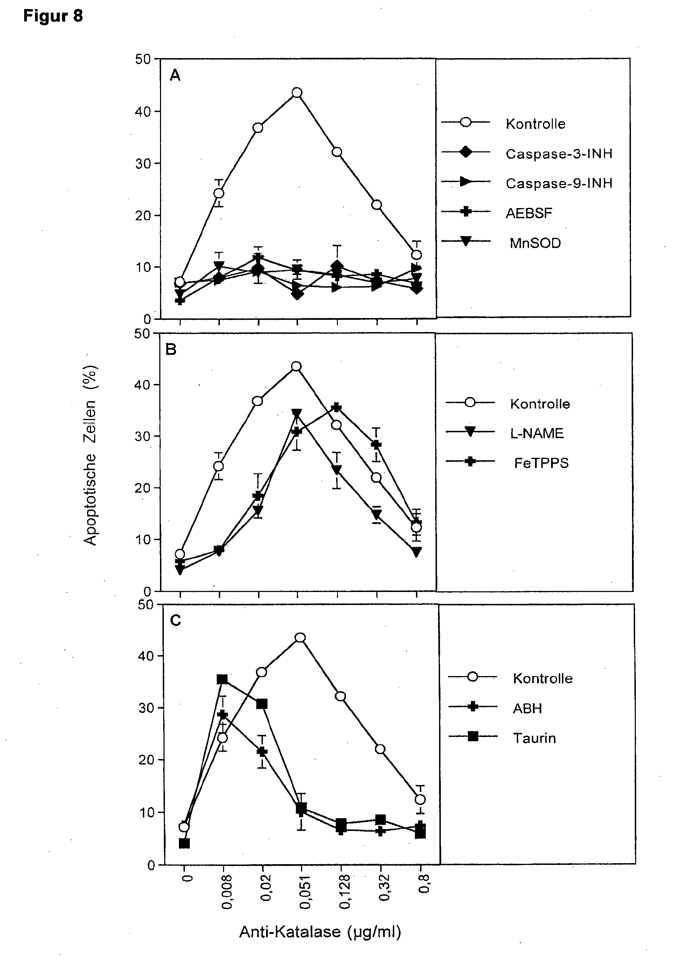

Fig. 8 zeigt, dass die Apoptoseinduktion in Tumorzellen durch Anti-Katalase-Antikörper auf der Wiederherstellung des interzellulären ROS Signaling beruht.

12 500 MKN-45 Zellen in 100 µl RPMI 1640 Medium, 10 % FBS wurden mit den angebenen Konzentrationen des monoklonalen Antikörpers gegen Katalase, sowie den genannten Inhibitoren versetzt und für 2 Stunden bei 37 °C, 5 % CO₂ weiterinkubiert, bevor in Doppelansätzen die Prozentsätze apoptotischer Zellen bestimmt wurden.

Inhibitoren: 50 µM Caspase-3-Inhibitor; 25 µM Caspase-9-Inhibitor, 100 µM AEBSF (NADPH-Oxidase-Inhibitor), 120 U/ml MnSOD, 2.4 mM L-NAME (NOS-Inhibitor); 40 µM FeTPPS (Peroxynitrite decomposition catalyst); 150 µM 4-Aminobenzoylhydrazid (ABH) (Peroxidase-Inhibitor); 50 mM Taurin (HOCI-Fänger).

Die Figur 8 zeigt, dass die Apoptoseinduktion in MKN-45-Zellen nach Applikation von monoklonalem Antikörper gegen Katalase durch Caspase-9 und Caspase-3 ausgeführt wird. Die Einleitung der Apoptose ist im gesamten Konzentrationsbereich des Antikörpers von Superoxidanionen abhängig, die im niedrigen Konzentrationsbereich des Antikörpers den NO/Peroxynitritweg, im höheren Konzentrationsbereich den HOCI-Weg antreiben.

Fig. 8 zeigt die Richtigkeit dieser Annahme und dass die Hemmung der Katalase durch Antikörper tatsächlich *funktionelle* Bedeutung für die Induktion der interzellulären Apoptose besitzt. Die konzentrationsabhängige Apoptoseinduktion in MKN-45-Zellen durch monoklonalen Antikörper gegen Katalase wird durch AEBSF (4-(2-aminoethyl)benzolsulfonylfluorid), einem Hemmstoff der Superoxidanionen-produzierenden NADPH-Oxidase und durch SOD gehemmt. Dies belegt, dass die Apoptoseinduktion im gesamten Konzentrationsbereich des Antikörpers von Superoxidanionen abhängig ist. Aufgrund der hemmenden Wirkung der nicht-zellpermeablen SOD wird klar, dass es sich um extrazelluläre Superoxidanionen handeln muss. Die vollständige Hemmung der Apoptose durch Caspase-9- und Caspase-3-Inhibitor beweist, dass der mitochondriale Weg der Apoptose abläuft. Die durch den Antikörper gegen Katalase induzierte Apoptose wird im niedrigen Konzentrationsbereich des Antikörpers durch L-NAME (N-w-nitro-L-arginiummethylester hydrochlorid), einem Inhibitor der NO-Synthase und durch FeTPPS (5-, 10-, 15-, 20-Tetrakis(4-sulfonatophenyl)porphyrinato iron(III) chloride), einem Peroxynitritzerstörer gehemmt, was auf die Rolle des NO/Peroxynitritwegs hinweist. Bei höherer Antikörperkonzentration wird der NO/Peroxynitritweg durch den HOCI-Weg abgelöst, wie aus der hemmenden Wirkung des Peroxidasehemmstoffs 4-Aminobenzoylhydrazid (ABH) und des HOCI-Fängers Taurin ersichtlich wird.

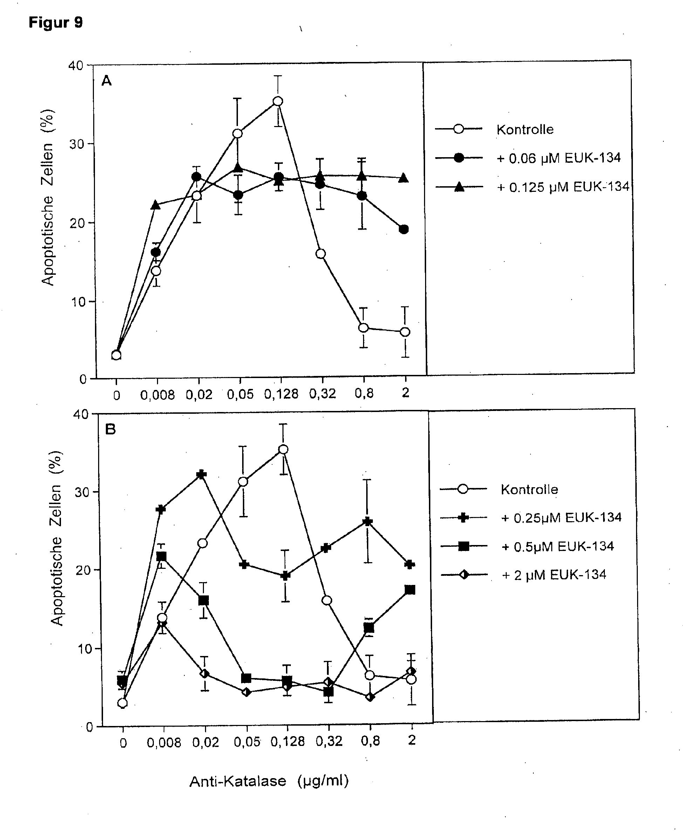

Fig. 9 erläutert die vielgestaltige Rolle von Wasserstoffperoxid beim interzellulären ROS Signaling.

12 500 MKN-45 Zellen in 100 µl RPMI 1640 Medium, 10 %FBS wurden mit den angebenen Konzentrationen der monoklonalen Antikörper gegen Katalase und den angegebenen Konzentrationen des Salen-Mangan-Komplexes EUK-134 (chloro[[2,2'-[1,2-ethanediylbis[(nitrilo-κN)methylidyne]]bis[6-meethoxyphenolato-κO]]]-manganese) (einem Katalasemimetikum) versetzt und für 2.5 Stunden bei 37 °C, 5 % CO₂ weiterinkubiert. Danach erfolgte die Bestimmung des Prozentsatzes apoptotischer Zellen (Doppelansätze) nach den klassischen Apoptosekriterien Membran-Blebbing, Kernkondensation und/oder Kernfragmentation.

Die Figur 9 zeigt, dass sehr niedrige Konzentrationen des Katalasemimetikums nur einen geringfügigen Einfluss auf das Signaling bis zum Optimumsbereich haben, den supraoptimalen Bereich aber sehr deutlich in Richtung Optimum bringen, da sie das die Konsumreaktion mit HOCI auslösende überschüssige Wasserstoffperoxid zerstören. Im mittleren und höheren Konzentrationsbereich von EUK-134 wird der NO/Peroxynitritweg zunächst begünstigt und dann wiederum inhibiert. Der HOCI-Weg wird durch die mittleren und höheren Konzentrationen EUK-134 schliesslich gehemmt.

Fig. 9 beleuchtet die Ursache für das Auftreten einer Optimumskurve bei der Apoptoseinduktion in Tumorzellen durch monoklonale Antikörper gegen Katalase. Unterschiedliche Konzentrationen des Salen-Mangankomplexes EUK-134 werden bei dieser Analyse eingesetzt. EUK-134 wirkt als Katalasemimetikum, kann aber auch Peroxynitrit zerstören (Ophoven S and Bauer G. Salen manganese complexes: sophisticated tools fort he analysis of intercellular ROS signaling pathways. Anticancer Res. 30: 3967-3980, 2010). Allerdings bedarf es für die Zerstörung des direkt an der Zellmembran von Tumorzellen nach Katalasehemmung entstehenden Peroxynitrits aus sterischen und kinetischen Gründen relativ hoher Konzentrationen von EUK-134. Daher haben niedrige Konzentrationen EUK-134 im Wesentlichen nur eine Wirkung auf Wasserstoffperoxid.

Fig. 9 zeigt, dass 0.06 und 0.125 µm Euk-134 die Optimumskurve in eine Plateaukurve umformen. Dies zeigt, dass der supraoptimale Abfall der Apoptoseinduktion durch überschüssiges Wasserstoffperoxid verursacht wird, von dem aus Bechtel W und Bauer G [(2009), Modulation of intercellular ROS signaling of human tumor cells, Anticancer Res. 29, 4559-4570; Heinzelmann S and Bauer G (2010), Multiple protective functions of catalase against intercellular apoptosis-inducing ROS signaling of human tumor cells, Biol. Chem. 391, 675-693] bekannt ist, dass es HOCI konsumieren und dadurch den HOCI-Weg zu Abbruch bringen kann. 0.06 und 0.125 µM EUK-134 heben diese Konsumreaktion offensichtlich auf, während sie die Apoptoseinduktion im Optimum in weitaus geringerem Maß reduzieren. Bei 0.25 µM EUK-134 ergibt sich ein deutlicher Anstieg der Apoptose im niedrigen Konzentrationsbereich der Antikörper, also dort, wie aufgrund der in Fig. 8 gezeigten Daten bevorzugt der NO/Peroxynitritsignalweg abläuft. Diese Stimulation des NO/Peroxynitritwegs kann durch die Aufhebung der Konsumreaktion zwischen Wasserstoffperoxid und NO erklärt werden. 0.25 µM EUK-134 führen im mittleren Konzentrationsbereich des Antikörpers erwartungsgemäß zu einer stärkeren Hemmung, da mehr Wasserstoffperoxid aus dem System entfernt wird und daher der HOCI-Weg gebremst wird. Im hohen Konzentrationsbereich des Antikörpers wird weiterhin die supraoptimale Wirkung von überschüssigem Wasserstoffperoxid aufgehoben und die Reaktion auf ein Plateau gebracht. Die beiden nächsthöheren Konzentrationen an EUK-134 führen dann zu einer vollständigen Hemmung des HOCI-Wegs im mittleren Konzentrationsbereich des Antikörpers, und erlauben Apoptoseinduktion erst bei sehr hohen Antikörperkonzentrationen. Gleichzeitig nimmt die stimulierende Wirkung des EUK-134 auf den NO/Peroxynitritweg wieder ab, da der Steigerung des Signalwegs durch Aufheben der Konsumreaktion zwischen NO und Wasserstoffperoxid nun die Zerstörung des gebildeten Peroxynitrits durch EUK-134 entgegenwirkt.

### Beispiel 2: Sensitivierung von Tumorzellen für interzelluläre Apoptoseinduktion durch die Wirkung von monoklonalen Antikörpern gegen SOD

Die publizierten Daten legen nahe, dass Katalase Tumorzellen vor allen interzellulären ROS-Signalwegen wirkungsvoll schützen kann und, dass dementsprechend eine Hemmung oder Inaktivierung der Katalase notwendig und ausreichend sein sollte, um diesen Schutz wieder aufzuheben und die Tumorzellen durch nachfolgend reaktiviertes interzelluläres ROS-Signaling in die Apoptose zu treiben.

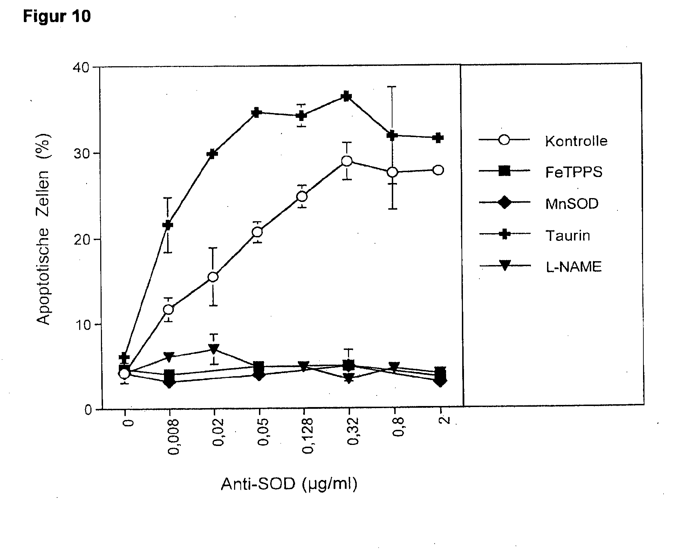

Figur 10 zeigt die spezifische Apoptoseinduktion in MKN-45-Zellen durch monoklonale Antikörper gegen SOD.

12 500 MKN-45 Zellen in 100 µl RPMI 1640 Medium, 10 %FBS wurden mit den angebenen Konzentrationen von monoklonalen Antikörpern gegen SOD und den angegebenen Inhibitoren versetzt und für 6 Stunden bei 37 °C, 5 % CO₂ weiterinkubiert. Danach erfolgte die Bestimmung des Prozentsatzes apoptotischer Zellen (Doppelansätze) nach den klassischen Apoptosekriterien Membran-Blebbing, Kernkondensation und/oder Kernfragmentation.

Inhibitoren: 25 µM FeTPPS (Peroxynitrite decomposition catalyst); 75 U/ml MnSOD; 50 mM Taurin (HOCI-Fänger); 2.4 mM L-NAME (NOS-Inhibitor).

Das Experiment zeigt, dass monoklonaler Antikörper gegen SOD in MKN-45-Zellen Apoptose induziert die ausschliesslich auf dem NO/Peroxynitritweg beruht. Der HOCI-Weg scheint unter diesen Bedingungen keine Rolle zu spielen. Der verstärkende Effekt des Taurins beruht darauf, dass das Wegfangen von HOCI durch Taurin die Umsetzung von Wasserstoffperoxid beschleunigt, so dass die Konsumreaktion zwischen Wasserstoffperoxid und NO gemindert und dadurch der NO/Peroxynitritweg verstärkt wird.

Daher ist der in Fig. 10 gezeigte Befund der Apoptoseinduktion in Tumorzellen durch monoklonale Antikörper gegen SOD unerwartet und bei erster Betrachtung zunächst schwer einzuordnen. Fig. 10 zeigt, dass die Zugabe von Anti-SOD zur Magenkarzinomzelllinie MKN-45 zu konzentrationsabhängiger Apoptoseinduktion führt, die von Superoxidanionen (Hemmung durch SOD), NO (Hemmung durch L-Name) und Peroxynitrit (Hemmung durch FeTPPS), also den klassischen Spielern des NO/Peroxynitritwegs abhängig ist, während die fehlende Hemmbarkeit durch Taurin darauf hinweist, dass der HOCI-Weg unter diesen Bedingungen keine Rolle spielt. Die steigernde Wirkung von Taurin auf den NO/Peroxynitritweg ist durch die Aufhebung der Konsumreaktion zwischen NO und Wasserstoffperoxid erklärbar. In Gegenwart von Taurin wird bei der hier offenbar geringfügig ablaufenden HOCI-Synthese durch Wegfangen des HOCI die Umsetzung von Wasserstoffperoxid durch Peroxidase beschleunigt und dadurch die Konsumreaktion von NO vermindert. Der Zuwachs von NO und dessen Einspeisung in den NO/Peroxynitritsignalweg schlagen anscheinend stärker zu Buch als der Verlust von HOCI.

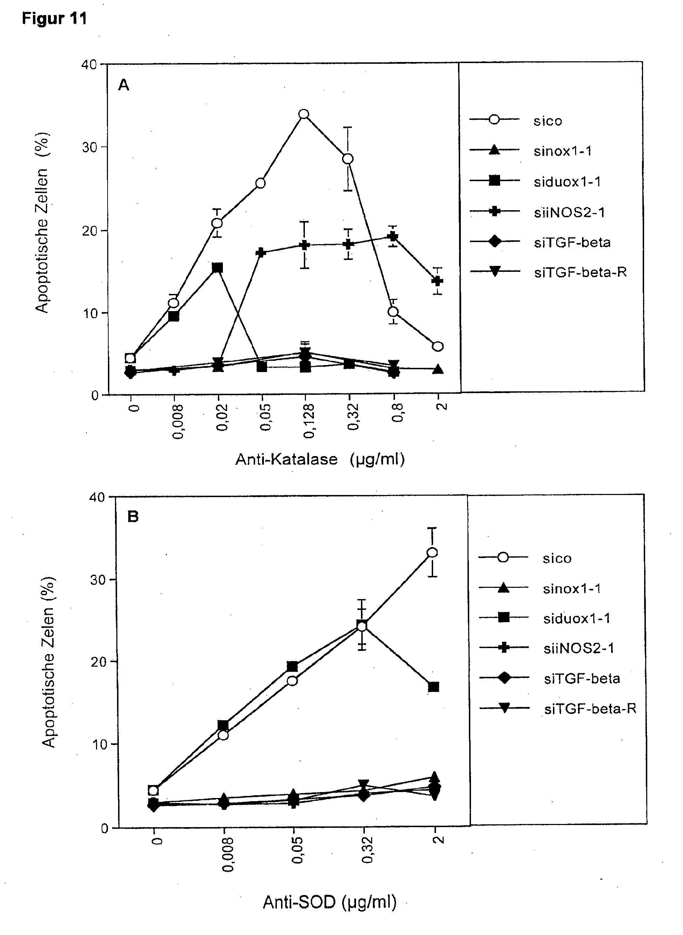

Die in Fig. 11 zusammengefassten Versuche zeigen eine SiRNA-gestützte Analyse der Signalkomponenten für die Apoptoseinduktion durch Anti-Katalase und Anti-SOD.

300 000 MKN-45-Zellen/ml wurden mit den angegebenen siRNAs (24 nM) transfiziert und nach 24 Stunden gewaschen und in frischem Medium ohne siRNA aufgenommen. Antikörper gegen Katalase oder SOD wurden in den angegebenen Konzentrationen appliziert. Nach 2.5 Stunden Inkubation wurden die Prozentsätze apoptotischer Zellen in Doppelansätzen bestimmt.

Das Experiment zeigt, dass siRNA gegen NOX-1, TGF-beta und TGF-beta-Rezeptor die Apoptoseinduktion durch Anti-Katalase und Anti-SOD sehr stark und über den gesamten Konzentrationsbereich hemmt. Apoptoseinduktion durch Anti-Katalase hängt von iNOS und Duox-1ab, während die Apoptoseinduktion nach Anti-SOD-Gabe im wesentlichen von iNOS bestimmt wird.

Fig. 11 belegt, dass bei der Apoptoseinduktion in MKN-45-Tumorzellen sowohl durch anti-Katalase als auch Anti-SOD die Signalkomponenten NOX-1, sowie TGF-beta und TGF-beta-Rezeptor von zentraler Bedeutung sind. Die Apoptoseinduktion durch Anti-Katalase beruht im niedrigen Konzentrationsbereich des Antikörpers vor allem auf der Wirkung von iNOS, während bei höheren Konzentrationen iNOS und Duox-1 zusammenwirken. Die Apoptoseinduktion durch Anti-SOD beruht hingegen fast vollständig auf NOX-1 und iNOS, wobei auch hier TGF-beta und TGF-beta-Rezeptor für die Wirkung unverzichtbar sind.

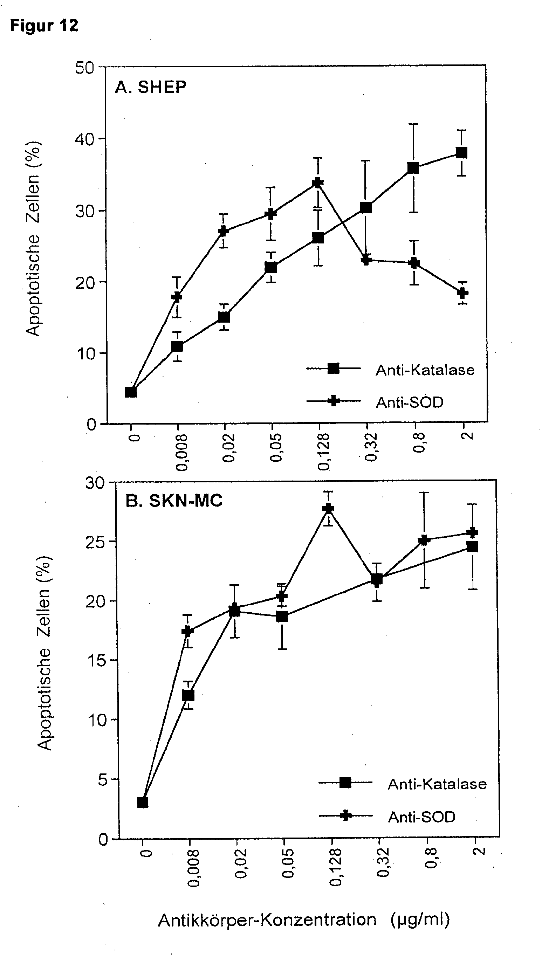

Fig. 12 zeigt, dass monoklonale Antikörper gegen Katalase und SOD Apoptose in den Zelllinien SHEP und SKN-MC induzieren.

12 500 Zellen der humane Neuroblastomlinie SHEP und der humanen Ewing-Sarkomlinie SKN-MC in 100 µl EMEM plus 5 % FBS wurden mit den angegebenen Konzentrationen Anti-Katalase bzw. Anti-SOD versetzt. Die Bestimmung der Prozentsätze apoptotischer Zellen erfolgte nach 5 Stunden (SHEP) bzw. 5.5 Stunden (SKNMC).

Das Ergebnis zeigt, dass Apoptoseinduktion durch Antikörper gegen Katalase oder SOD nicht nur auf die Zellinie MKN-45 beschränkt ist, sondern breit anwendbar ist.

Parallele Kontrollen mit Kontroll-IgG ergaben keine Apoptoseinduktion (Daten nicht gezeigt).

Fig. 12 belegt die Allgemeingültigkeit des neuen und unerwarteten Befundes, nämlich dass sowohl anti-Katalase als auch anti-SOD in der Lage sind, Apoptose in Tumorzellen zu induzieren. Anhand der Neuroblastomlinie SHEP und der Ewingsarcomlinie SKNMC wird demonstriert, dass sowohl anti-Katalase als auch anti-SOD zur Apoptoseinduktion führt, die von der Konzentration der jeweiligen Antikörper direkt abhängig ist.

In Fig. 13 wird gezeigt, dass Apoptoseinduktion in SKNMC-Zellen durch Anti-Katalase und Anti-SOD durch ROS-Signaling vermittelt wird.

10 000 Zellen der humanen Ewing-Sarkomlinie SKN-MC in 100 µl EMEM plus 5 % FBS wurden mit den angegebenen Inhibitoren und den in der Abszisse aufgetragenen Konzentrationen Anti-Katalase bzw. Anti-SOD versetzt. Die Bestimmung der Prozentsätze apoptotischer Zellen erfolgte nach 3 Stunden.

Inhibitoren: 50 mM Taurin (HOCI-Fänger); 40 µM FeTPPS (Peroxynitrite decomposition catalyst); 1000 U/ml Katalase aus Rinderleber; 40 µM MnTE2PyP (SOD-Mimetikum); 90 U/ml MnSOD; 20 µM 3-Br-7-Nitroindazol (nNOS-spezifischer Inhibitor).

Die Figur belegt, dass die Ewingsarkomlinie SKNMC sowohl durch anti-Katalase als auch anti-SOD zum interzellulären ROS-Signaling ausschliesslich über den NO/Peroxynitritweg sensitiviert wird. Der HOCI-Weg scheint keine Rolle zu spielen, wie aus der fehlenden Hemmbarkeit durch den HOCI-Fänger Taurin ersichtlich wird. Bemerkenswert und wichtig ist auch der Befund, dass die Wirkung von anti-SOD durch exogene Katalase aufgehoben werden kann, was darauf hinweist, dass anti-SOD auch zu einer Hemmung von Tumorzellkatalase führen könnte.

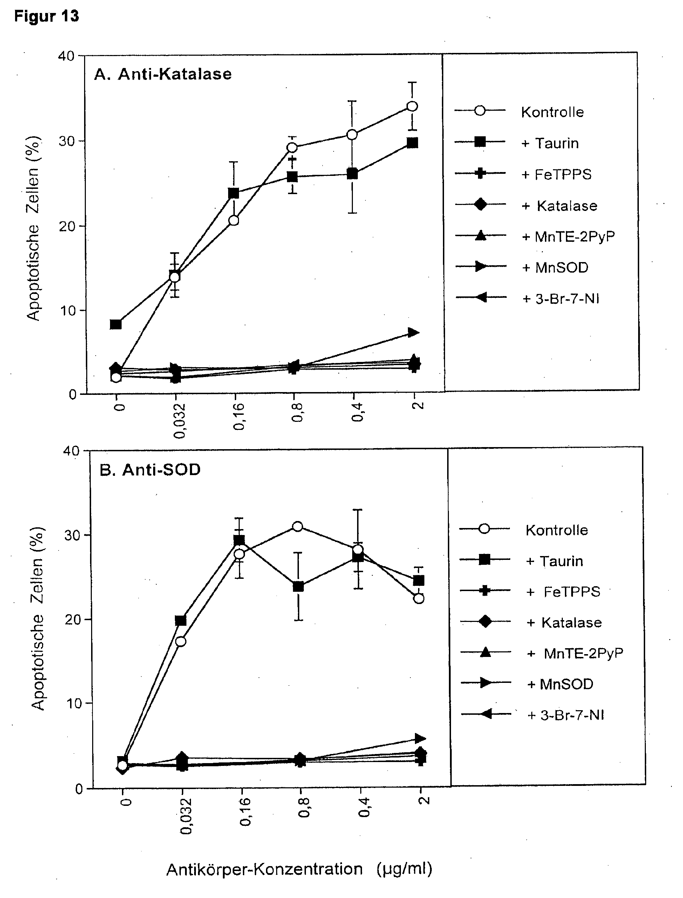

Fig. 13 zeigt anhand der Zelllinie SKN-MC, dass sowohl anti-Katalase als auch anti-SOD die Zellen für interzelluläres ROS-Signaling mithilfe des NO/Peroxynitritsignalwegs sensitivieren, da eine Abhängigkeit von Superoxidanionen (Hemmung durch SOD und das SOD-Mimetikum MnTE2PyP), NO (Hemmung durch den nNOS-spezifischen Inhibitor 3-Br-7-Nitroindazol) und deren Reaktionsprodukt Peroxynitrit (Hemmung durch FeTPPS) nachgewiesen wird. Der HOCI-Weg scheint hier keine erkennbare Rolle zu spielen, was an der fehlenden Hemmwirkung von Taurin erkennbar ist. Die Hemmung durch die hohe Konzentration exogen zugebener Katalase ist in Einklang mit der Annahme, dass hohe Konzentrationen exogener Katalase in der Lage sind, an der Zellmembran durch die Zellen selbst gebildetes Peroxynitrite zu entgiften, bevor dieses durch Lipidperoxidation Apoptose induziert. Die gleiche Funktion kann von membranständiger Katalase der Tumorzellen durchgeführt werden. Der Befund, dass in diesem Zellsystem, das ausschliesslich Signaling über den NO/Peroxynitritweg ausführt, der Effekt von Anti-Katalase durch exogene Katalase wieder aufgehoben werden kann ist schlüssig und als Kontrolle auch zu fordern. Dass aber die Wirkung von anti-SOD (bei der theoretisch kein Einfluss auf den ausreichend schützenden Katalasegürtel auf der Aussenmembran der Zelle zu erwarten ist) ebenfalls durch hohe Katalasekonzentrationen aufgehoben wird weist nun erstmals darauf hin, dass die Hemmung der SOD auch einen negativen Einfluss auf die protektive Katalase der Tumorzellen haben muss.

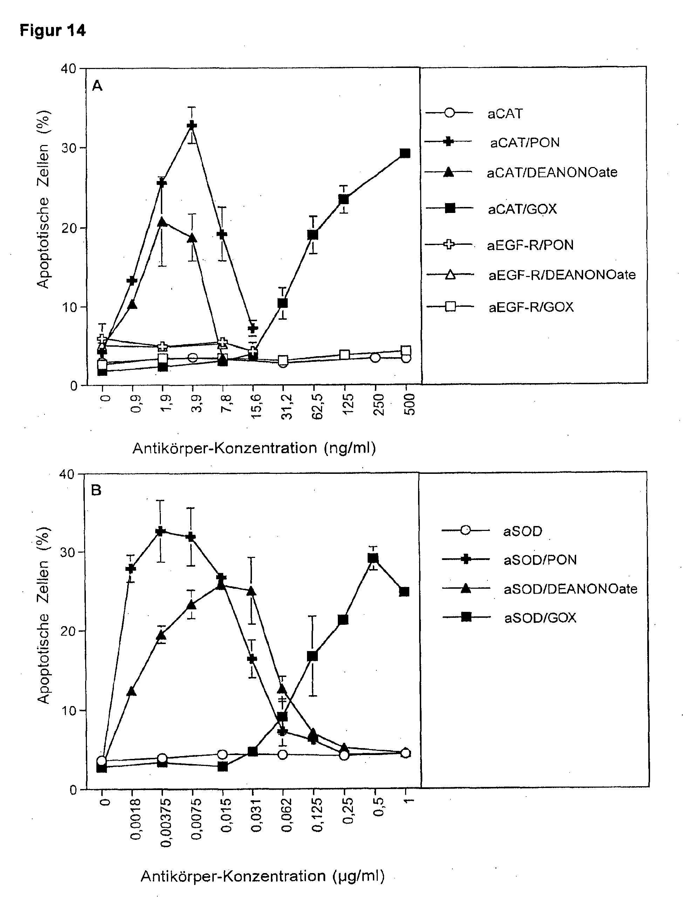

Die Fig. 14 belegt, dass Anti-Katalase- und Anti-SOD-Antikörper zu einer Hemmung der Tumorzellkatalase führen.

4000 MKN-45-Zellen in 100 µl RPMI 1640 Medium, 10 %FBS wurden mit den angegebenen Konzentrationen Anti-Katalase bzw. Anti-SOD, und als Kontrolle Anti-EGF-Rezeptor versetzt. Nach 15 Minuten erfolgte die Zugabe von entweder 200 µM Peroxynitrit (PON), 0.5 mM des NO-Donors DEA-NONOate oder 2 mU/ml Glukoseoxidase (Wasserstoffperoxidgenerator). Nach 2 Stunden Inkubation bei 37 °C, 5 % CO₂ wurden die Prozentsätze apoptotischer Zellen in Doppelansätzen bestimmt.

Diese Figur zeigt eindrucksvoll, dass sowohl anti-Katalase als auch anti-SOD, nicht aber irrelevanter Kontrollantikörper, zu einer Hemmung der Katalaseaktivität der Tumorzellen führen, da diese nun für die Katalasesubstrate Peroxynitrit (entweder direkt zugegeben oder durch die Interaktion von NO aus dem NO-Donor und von Zellen generierten Superoxidanionen gebildet) und Wasserstoffperoxid empfindlich werden. Die Optimumskurve bei der Peroxynitritwirkung ist dadurch erklärbar, dass bei steigender Katalasehemmung zelleigenes Wasserstoffperoxid zu einem Verbrauch von Peroxynitrit führt.

In Fig. 14 wird gezeigt, dass sowohl die Zugabe von Antikörpern gegen Katalase als auch gegen SOD zu einer Sensitivierung von MKN-45-Tumorzellen gegen die Apoptoseauslösende Wirkung von Peroxynitrit und Wasserstoffperoxid führt. Peroxynitrit wird in diesem Experiment sowohl direkt zugegeben, als auch nach Applikation des NO-Donors DEA-NONOate an der Zellmembran durch die Interaktion von aus dem NO-Donor freigesetztem NO mit von NOX-1 gebildeten Superoxidanionen gebildet. Direkt Apoptoseauslösende Wasserstoffperoxidkonzentrationen werden durch Glucoseoxidase generiert. Fig. 14 zeigt, dass sowohl Anti-Katalase als auch Anti-SOD die Zellen gegen Peroxynitrit und Wasserstoffperoxid sensitivieren. Die Sensitivierung gegen Peroxynitrit erfolgt im niedrigeren Konzentrationsbereich der Antikörper und zeigt die Form einer Optimumskurve. Wie in Heinzelmann and Bauer 2010 gezeigt, wird der abfallende Teil der Optimumskurve durch den Konsum von Peroxynitrit durch Wasserstoffperoxid bedingt. Die Sensitivierung gegen exogen generiertes Wasserstoffperoxid benötigt höhere Konzentrationen der Antikörper. Kontrollantikörper, die gegen den membranständigen EGF-Rezeptor gerichtet sind zeigten keinerlei sensitivierende Wirkung. Die in Fig. 14 gezeigten Befunde stellen eine Herausforderung an die Interpretationsfähigkeit des Experimentators dar. Unter der Annahme, dass Tumorzellen allein durch Katalase vor dem interzellulären Signaling geschützt sind, wird die Sensitivierung der Zellen für NO, Peroxynitrit und Wasserstoffperoxid durch Anti-Katalase erwartet, die Sensitivierung durch anti-SOD ist jedoch unerwartet. Unter der Annahme, dass die Zellen sowohl Katalase als auch SOD auf ihrer Membran tragen ergibt sich für den Schutz der Zellen vor ROS eine ausgezeichnete Kooperationsmöglichkeit und gegenseitige Wirkungsverstärkung der beiden Enzyme, wie in Fig. 2 dargestellt wird. Katalase würde demnach durch die Zerstörung von Wasserstoffperoxid die HOCI-Synthese unterbinden und durch den Abbau von Peroxynitrit den NO/Peroxynitritweg unterbrechen. SOD würde die Bildung von Peroxynitrit unterbinden und zusätzlich die Interaktion von HOCI und Superoxidanionen verhindern. Jeder der beiden Signalwege wäre also durch die Interaktion von SOD und Katalase an zwei unterschiedlichen Stellen gehemmt.

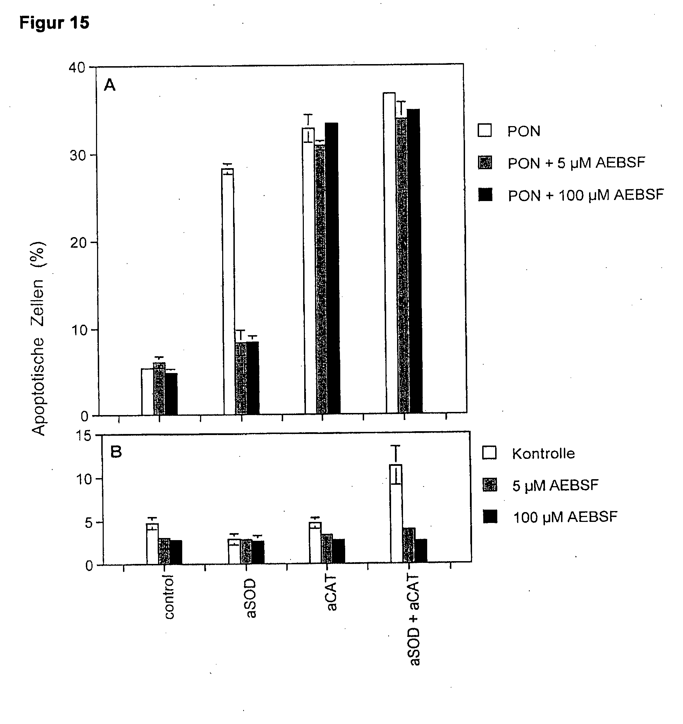

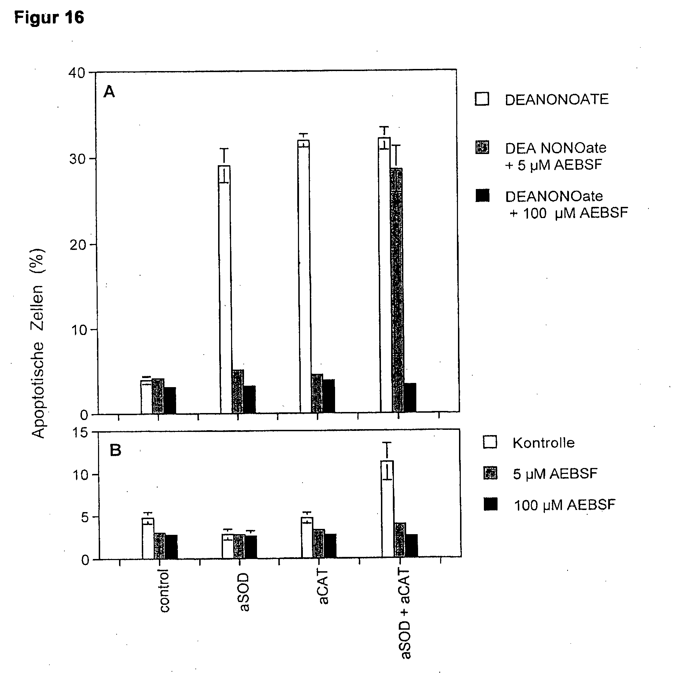

Betrachtet man unter dem Blickwinkel der Figuren 2 und 3 das in Fig. 14 vorgestellte Ergebnis, so bleibt die Sensitivierung der Zellen für exogen zugegebenes Peroxynitrit und für Wasserstoffperoxid durch Anti-Katalase weiterhin schlüssig, die Sensitivierung für Peroxynitrit, das aus NO und zelleigenen Superoxidanionen gebildet wird jedoch nicht, da hier, entsprechend der in der Schemazeichnung vorgestellten biochemischen Zusammenhänge, die SOD weiterhin durch Wegfangen der Superoxidanionen die Peroxynitritbildung unterbinden sollte. Die Hemmung der SOD durch Anti-SOD sollte zwar die Bildung von Peroxynitrit aus NO und Superoxidanionen erlauben (siehe Fig. 4), doch sollte dann die nicht gehemmte Katalase das Peroxynitrit abbauen. Aus einer einfachen Hemmung der SOD ist auch nicht zu erklären, auf welchem Weg die Zelle durch anti-SOD gegen exogenes Peroxynitrit und Wasserstoffperoxid sensitiviert wird. Die in Fig. 14 gezeigten Ergebnisse sind sofort in allen Punkten schlüssig zu erklären, wenn angenommen wird, dass die Verabreichung von anti-Katalase-Antikörpern auch zu einer Inaktivierung von SOD und die Verabreichung von anti-SOD-Antikörpern auch zu einer Inaktivierung der Katalase führt. Aufgrund der monoklonalen Natur der beiden Antikörperpräparationen und der Spezifitätsprüfung durch den Hersteller ist weder Kreuzkontamination noch Kreuzreaktion geeignet, diese Befunde zu erklären. Die folgende Figuren 15 und 16 lösen diese wichtige Frage auf.

Fig. 15 belegt die Bedeutung von Superoxidanionen für die Sensitivierung von Tumorzellen gegen exogenes Peroxynitrit durch Anti-SOD und Anti-Katalase.

4000 MKN-45-Zellen in 100 µl RPMI 1640 Medium, 10 % FBS blieben zunächst unbehandelt oder erhielten 5µM bzw. 100 µM AEBSF (Hemmstoff der NADPH-Oxidase). Anschliessend wurden entweder keine Antikörper ("control"), 10 ng/ml Anti-SOD ("aSOD"), 5 ng/ml Anti-Katalase ("aCAT") oder eine Kombination von Anti-SOD und Anti-Katalase ("aSODS + aCAT") zugegeben. Nach weiteren 15 Minuten erfolgte die Zugabe von 100 µM Peroxynitrit (PON) (A). Kontrollansätze (B) blieben frei von Peroxynitrit. Nach 2 Stunden wurden in Doppelansätzen die Prozentsätze apoptotischer Zellen bestimmt.

Die Figur 15 zeigt, dass Katalasehemmung der Tumorzellen durch Anti-Katalase auch dann erfolgreich abläuft, wenn die NADPH-Oxidase teilweise oder ganz gehemmt wird. Die Wirkung von anti-SOD auf die Tumorzellkatalase, die sich in einer Sensitivierung für Peroxynitrit ausdrückt ist jedoch von optimal laufender Superoxidanionenproduktion abhängig. Dies zeigt zunächst, dass anti-SOD keine direkte Hemmwirkung für Katalase besitzt, denn sonst sollte ihr Effekt in bezug auf fehlende Hemmbarkeit durch AEBSF dem von Anti-Katalase gleichen. Das Ergebnis zeigt auch, dass anti-SOD einen durch Superoxidanionen (oder deren Folgeprodukte) vermittelten indirekten Effekt auf die Tumorzellkatalase ausüben muss.

Fig. 15 zeigt, dass sowohl anti-Katalase-Antikörper als auch anti-SOD-Antikörper MKN-45 Zellen für die Wirkung von exogen zugegebenem Peroxynitrit sensitivieren. Erfolgt die jeweilige Antikörpergabe nachdem die Zellen mit AEBSF, einem Hemmstoff der NADPH-Oxidase behandelt wurden, so zeigt anti-Katalase weiterhin erwartungsgemäss eine sensitivierende Wirkung. Die Wirkung von Anti-SOD-Antikörpern auf die Sensitivierung gegen Peroxynitrit, einem Prozess, der sich nur durch gleichzeitige Aufhebung der Katalaseaktivität der Tumorzellen erklären lässt bleibt jedoch in Gegenwart von AEBSF vollständig aus. Dies bedeutet, dass die Wirkung von aSOD der Synthese von Superoxidanionen oder der Wirkung ihrer Folgeprodukte bedarf.

Fig. 16 erläutert die Bedeutung von Superoxidanionen für die Anti-Katalase- und Anti-SOD-abhängige Sensitivierung von Tumorzellen gegen Peroxynitrit, das durch die Interaktion eines exogenen NO-Donors mit extrazellulären Superoxidanionen der Tumorzelle gebildet wird gegen exogenes Peroxynitrit durch Anti-SOD und Anti-Katalase.

4000 MKN-45-Zellen in 100 µl RPMI 1640 Medium, 10 % FBS blieben zunächst unbehandelt oder erhielten 5µM bzw. 100 µM AEBSF (Hemmstoff der NADPH-Oxidase). Anschliessend wurden entweder keine Antikörper ("control"), 10 ng/ml Anti-SOD ("aSOD"), 5 ng/ml Anti-Katalase ("aCAT") oder eine Kombination von Anti-SOD und Anti-Katalase ("aSOD + aCAT") zugegeben. Nach weiteren 15 Minuten erfolgte die Zugabe von 0.5 mM DEA-NONOate, einem schnell zerfallenden NO-Donor (A). Kontrollansätze blieben frei von DEA-NONOate (B)Nach 2 Stunden wurden in Doppelansätzen die Prozentsätze apoptotischer Zellen bestimmt.

Für das Verständnis dieses Ergebnisses ist es wichtig, zunächst zu rekapitulieren, dass nach Freisetzung von NO aus DEA-NONOate dieses mit Superoxidanionen der Zelle zu Peroxynitrit reagiert, welches dann die Apoptose induziert. Daher ist dieser Prozess sowohl durch SOD als auch Katalase blockierbar. Da aus den bisher gezeigten Daten erkennbar ist, dass die Tumorzellen sowohl Katalase als auch SOD an ihrer Oberfläche tragen, lässt der Befund, dass sowohl anti-SOD als auch anti-Katalase zu einer Sensitivierung für den DEA-NONOate-Effekt führen den Schluss zu, dass die Behandlung mit anti-SOD auch die Katalase, die Behandlung mit anti-Katalase auch die SOD inaktivieren muss. In Gegenwart von 5 µM AEBSF scheint diese Wechselbeziehung nicht mehr zu funktionieren, da unter diesen Bedingungen die Wirkung von alleiniger Gabe von Anti-SOD oder Anti-Katalase, nicht aber die Wirkung der Kombination beider Antikörper unterbunden ist. Das Funktionieren der Kombination der Antikörper in Gegenwart von 5 µM AEBSF zeigt, dass hier noch ausreichend Superoxidanionen zur Peroxynitritbildung zur Verfügung stehen, während dies bei 100 µM AEBSF sicher nicht mehr der Fall ist. Der Befund ist insgesamt am besten dadurch zu erklären, dass anti-SOD in einem Superoxidanionen-abhängigen Prozess auch Katalase hemmt, umgekehrt anti-Katalase in einem Superoxidanionen-abhängigen Prozess SOD hemmt, so dass die Gabe eines der beiden Antikörper ausreicht, um den Gesamtprozess ablaufen zu lassen. In Gegenwart von 5 µM AEBSF bleibt die wechselseitige Hemmung aus, daher müssen nun beide Antikörper anwesend sein, um eine Sensitivierung zu erreichen.

Fig. 16 erweitert das Verständnis der Wechselbeziehungen zwischen anti-SOD und anti-Katalase. In diesem Experiment wird die Bildung des Apoptoseinduktors Peroxynitrit durch die Gabe des exogenen NO-Donors DEA-NONOate und die Verfügbarkeit zellulärer Superoxidanionen ermöglicht. Auch hier führt die alleinige Applikation von entweder anti-Katalase oder anti-SOD jeweils zu einer Sensitivierung der Zellen für Apoptoseinduktion. Sowohl die teilweise Hemmung der Superoxidanionenproduktion durch 5 µM AEBSF als auch deren vollständige Hemmung durch 100 µM AEBSF verhindert die apoptoseauslösende Wirkung von anti-Katalase-Antikörpern und von anti-SOD-Antikörpern. In Gegenwart von 100 µM AEBSF erfolgt bei gleichzeitiger Gabe der beiden Antikörper keine Apoptoseinduktion, was durch das Ausbleiben der Peroxynitritbildung bei vollständig unterbundener Superoxidanionensynthese auch zu fordern ist. In Gegenwart von 5 µM AEBSF wird jedoch die Apoptoseinduktion durch gleichzeitige Applikation der beiden Antikörper nicht signifikant gehemmt, während die sensitivierende Wirkung der Gabe von anti-Katalase oder anti-SOD allein in Gegenwart von 5 µM AEBSF vollständig aufgehoben wird. Dies zeigt, dass in Anwesenheit von 5 µM AEBSF noch ausreichend Superoxidanionen zur Peroxynitritbildung vorhanden sind, den sonst könnte bei gleichzeitiger Gabe von anti-SOD und Anti-Katalase nicht die beobachtete Apoptoseinduktion erfolgen. Das Ergebnis zeigt auch, dass Apoptoseinduktion durch Peroxynitrit, das im Ansatz nach Interaktion von NO und Superoxidanionen gebildet wird sowohl durch SOD als auch durch Katalase gehemmt wird, wie dies aus Fig. 2 auch zu erwarten ist. Daher ist auch der Schluss möglich, dass die Gabe von Anti-SOD-Antikörpern auch zu einer Hemmung der Katalase, die Gabe von anti-Katalase-Antikörpern auch zu einer Hemmung von SOD führt, und dass diese zusätzlichen Hemmungen des jeweils anderen Targets der Anwesenheit von Superoxidanionen oder deren Produkte bedürfen. Es liegt nahe, anzunehmen, dass die bei Hemmung der Katalase lokal in hoher Konzentration vorliegenden Superoxidanionen zu einer Hemmung der Katalase führen und das bei Hemmung der Katalase auftretende Wasserstoffperoxid in hoher lokaler Konzentration zu einer Substrathemmung der SOD führt.

### Beispiel 3: Synergistische Wirkung zwischen anti-Katalase und anti-SOD

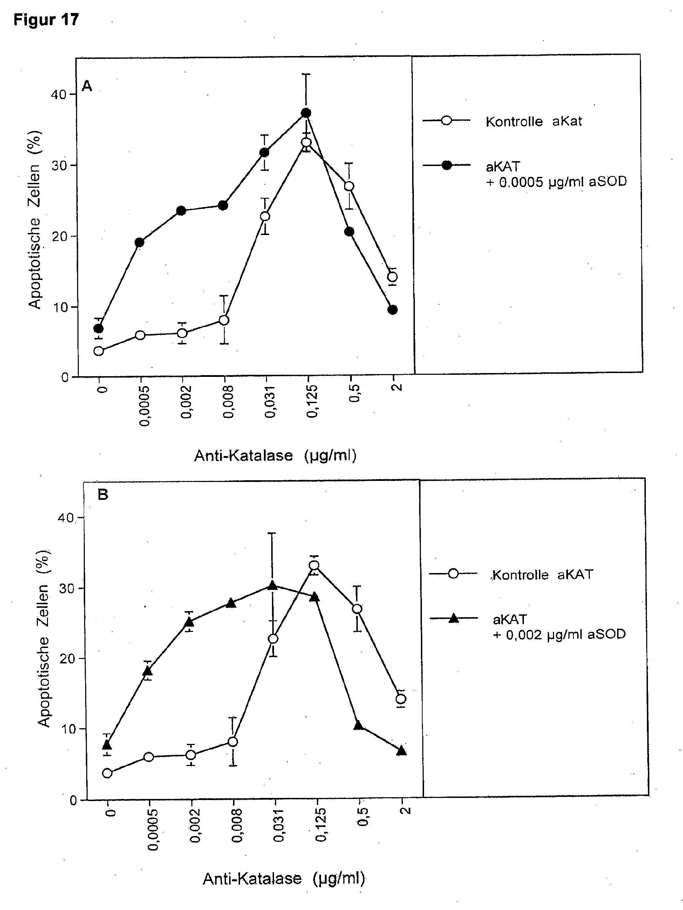

Fig. 17 zeigt eine synergistische Wirkung von Antikörpern gegen SOD und Katalase bei der ROS-vermittelten Apoptoseinduktion in Tumorzellen

12 500 MKN-45-Zellen in 100 µl RPMI 1640 Medium, 10 %FBS erhielten die angegebenen Konzentrationen anti-Katalase. Kontrollansätze blieben frei von weiteren Ansätzen, Ansätze zur Bestimmung der Interaktion erhielten entweder 0.5 ng/ml oder 2 ng/ml Anti-SOD zusätzlich. Die Bestimmung der Prozentsätze apoptotischer Zellen erfolgte nach 5.5 Stunden in Doppelansätzen.

Das Ergebnis zeigt einen sehr ausgeprägten synergistischen Effekt beim Zusammenwirken von Anti-SOD und Anti-Katalase.

Fig. 17 zeigt zunächst die Wirkung von Antikörpern gegen Katalase, die zu einer konzentrationsabhängigen Apoptoseinduktion in Form einer Optimumskurve führt. Bei gleichzeitiger Zugabe von sehr geringen Konzentrationen aSOD, die für sich allein eine kaum erkennbare Apoptoseinduktion herbeiführen, ergibt sich ein sehr ausgeprägter synergistischer Effekt. Dieser ist unerwartet und von grossem Interesse, da auf seiner Basis eine beachtliche Einsparung von Antikörpern bei gleicher Wirkung erzielt werden könnte. So führt beispielsweise die Kombination von 2 ng/ml Anti-SOD mit mit 2 ng/ml anti-Katalase zu 80 Prozent der Wirkung, die durch 125 ng/ml anti-Katalase-Antikörper allein erreicht werden kann.

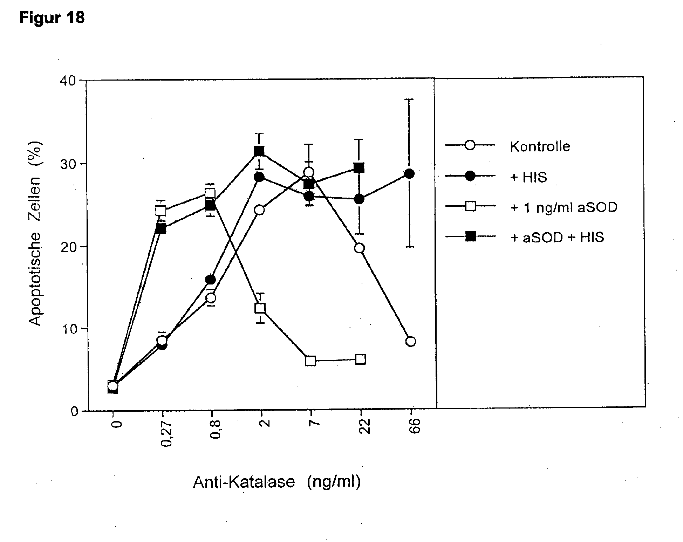

Fig. 18 belegt, dass der synergistische Effekt zwischen Anti-SOD und Anti-Katalase nicht durch Singulettsauerstoff vermittelt wird.

12 500 MKN-45-Zellen in 100 µl RPMI 1640 Medium, 10 %FBS erhielten die angegebenen Konzentrationen anti-Katalase (Kontrolle), anti-Katalase plus 2 mM Histidin (Singulettsauerstofffänger), anti-Katalase plus 1 ng/ml anti-SOD, anti-Katalase/1 ng/ml anti-SOD/2 mM Histidin. Die Bestimmung der Prozentsätze apoptotischer Zellen erfolgte nach 3 Stunden in Doppelansätzen.

Die Figur zeigt, dass der synergistische Effekt zwischen Anti-SOD und Anti-Katalase nicht durch Singulettsauerstoff bewirkt wird. Allerdings wird jeweils die supraoptimale Hemmung durch Histidin aufgehoben, was auf eine Rolle von Singulettsauerstoff in diesem Konzentrationsbereich hinweist.

Fig. 18 zeigt, dass dem synergistischen Effekt zwischen anti-SOD und anti-Katalase keine Singulettsauerstoffwirkung zugrunde liegt, da der Effekt nicht durch den Singulettsauerstofffänger Histidin blockiert werden kann. Es zeigt sich in diesem Experiment der wichtige Befund, dass der supraoptimale Abfall der Konzentrationsabhängigkeits-Kurven jeweils durch Histidin blockiert werden kann. Als Erklärungsmöglichkeit bietet sich aufgrund der bekannten Signalwege an, dass es im supraoptimalen Konzentrationsbereich der Antikörper aufgrund der Bildung von Singulettsauerstoff zu einer Inaktivierung von Katalase kommt, wodurch dann schliesslich die verfügbare Konzentration an Peroxidase nicht mehr ausreicht, alles Wasserstoffperoxid in HOCI umzusetzen. Dadurch kann dann die in Fig. 9 vorgestellte und durch Bechtel und Bauer, 2009, genauer analysierte Konsumreaktion zwischen HOCI und Wasserstoffperoxid den HOCI-Weg zum Abbruch bringen.

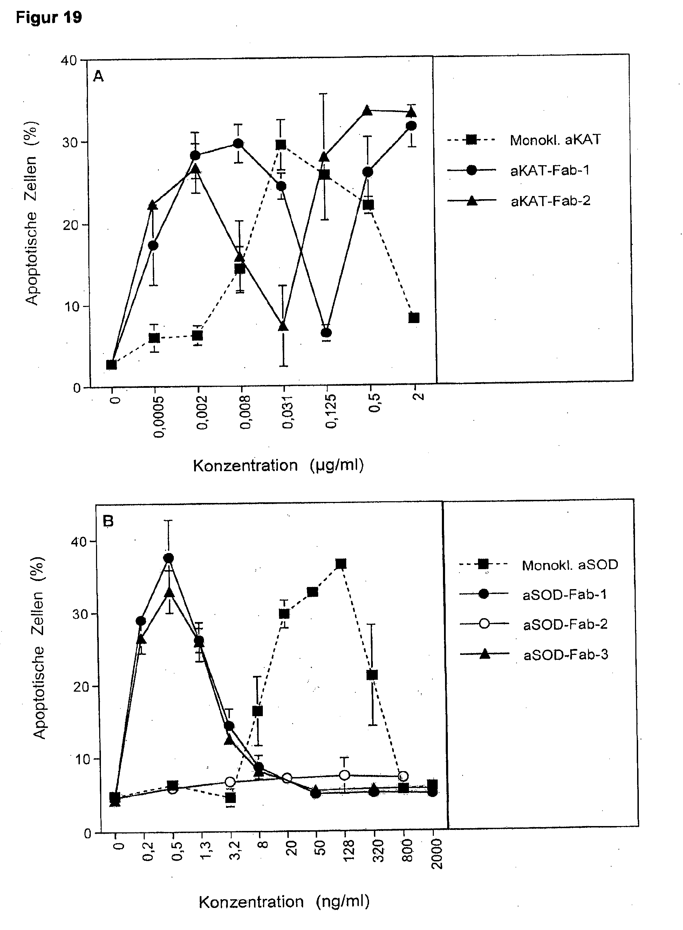

Fig. 19 erläutert die Sensitivierung der humanen Tumorzelllinie MKN-45 durch humanisierte Fab-Fragmente gegen Katalase bzw. SOD.

12 500 MKN-45-Zellen in 100 µl RPMI 1640 Medium, 10 %FBS erhielten im Versuchsteil A die angegebenen Konzentrationen anti-Katalase (monoklonal), humanisierte Fab-Fragmente gegen Katalase (AbD 15563, AbD 15562), im Versuchsteil B die angegebenen Konzentrationen anti-SOD (monoklonal), humanisierte Fab-Fragmente gegen SOD (AbD 15660, AbD15661, AbD15662). Die Doppelansätze in Teil A wurden nach 6 Stunden, die in Versuchsteil B nach 3 Stunden bewertet.

Die Figur 19 zeigt, dass humanisierte Fab-Fragmente gegen Katalase oder SOD ebenso wie monoklonale Antikörper gegen die gleichen Zielstrukturen eine starke sensitivierende Wirkung auf humane Tumorzellen haben. Dies weist darauf hin, dass die spezifische Bindung der Antikörper bzw. Fab-Fragmente und die daraus resultierende Hemmung bedeutsam für die beobachtete Apoptoseinduktion sind. Die Figur 19 zeigt auch, dass zwischen Fab-Fragmenten, die an die Zielstruktur binden und solchen, die deren Funktion auch inhibieren differenziert werden kann. So binden alle drei vorgestellten Fab-Fragmente gegen SOD an dieses Enzym, während nur Fab-1 (AbD 15660) und Fab-3 (AbD 15662) diese auch inaktivieren und dadurch ROS-Signaling initiieren.

Fig. 19 zeigt, dass die Wirkung des monoklonalen Antikörpers gegen Katalase auch mithilfe von humanisierten Fab-Fragmenten erzielt werden kann. Bei der hier gewählten Auftragung der Konzentrationen in µg/ml ist dabei erwartungsgemäß eine größere Wirkung der Fab-Fragment zu beobachten. Die Konzentrationsabhängigkeit der Wirkung der Fab-Fragmente stellt sich als Optimumskurve dar. Parallel durchgeführte Inhibitorexperimente belegen, dass es sich im Bereich der Optimumskurve initial um den NO/Peroxynitritweg handelt, der dann vom HOCI-Weg abgelöst wird, bevor aufgrund exzessiver Wasserstoffperoxidkonzentrationen dieser durch die vorher diskutierte Verbrauchsreaktion gehemmt wird. Bei einer weiteren Zugabe von Fab gegen Katalase im Bereich ab 0.5 µg/ml kommt es dann zu einer direkten Apoptoseinduktion durch Wasserstoffperoxid.

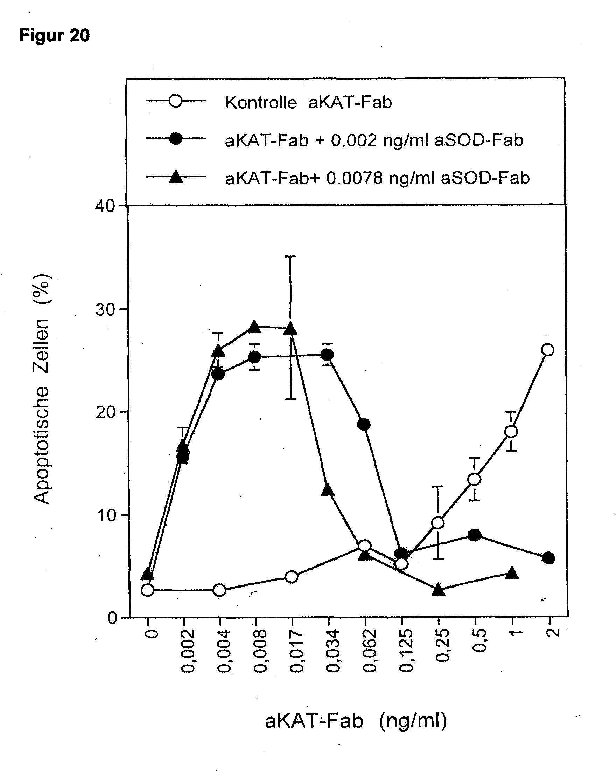

Fig. 20 zeigt die synergistische Wirkung von Fab-Fragmenten gegen SOD und Katalase bei der ROS-vermittelten Apoptoseinduktion in Tumorzellen.

12 500 MKN-45-Zellen in 100 µl RPMI 1640 Medium, 10 %FBS erhielten die angegebenen Konzentrationen des gegen Katalase gerichteten Fab-Fragments AbD15562. Kontrollansätze blieben frei von weiteren Ansätzen, Ansätze zur Bestimmung der Interaktion erhielten entweder 0.002 ng/ml oder 0.0078 ng/ml des Anti-SOD Fab-Fragments AbD 15660 zusätzlich. Die Bestimmung der Prozentsätze apoptotischer Zellen erfolgte nach 3 Stunden in Doppelansätzen.

Dieses Ergebnis zeigt, dass auch mit Fab-Fragmenten die gegen Katalase bzw. SOD gerichtet sind ein bemerkenswerter synergistischer Effekt bei der Apoptoseinduktion in Tumorzellen erreicht werden kann. Die dafür benötigten beeindruckend niedrigen Konzentrationen an Fab-Fragmenten ermutigen zu der Hoffnung, daraus einen finanzierbaren Therapieansatz entwickeln zu können.

Fig. 20 zeigt, dass sich auch gegen SOD humanisierte Fab-Fragmente darstellen lassen, die mit grosser Effizienz Apoptose in menschlichen Tumorzellen induzieren. Auch hier ist die erwartete höhere Wirksamkeit im Vergleich zu monoklonalen Antikörpern zu beobachten.

Schließlich zeigt Fig. 20, dass die Kombination von humanisierten Fab-Fragmenten gegen Katalase mit humanisierten Fab-Fragmenten gegen SOD zu einem beachtlichen synergistischen Effekt führt. So zeigt die Kombination von 8 pg/ml Anti-Katalase mit 8 pg/ml anti-SOD den gleichen Effekt wie 2 ng/ml Anti-Katalase allein.

Aufgrund von Publikationen war bekannt, dass Katalase eine hervorragende und zentrale Rolle bei der Hemmung der interzellulären ROS-vermittelten Signalwege von Tumorzellen besitzt und dabei sowohl den HOCI- als auch den NO/Peroxynitritsignalweg inhibiert. Hemmung oder Inaktivierung der Katalase wurde bereits als Weg zur spezifischen Apoptoseinduktion in Tumorzellen erkannt.

Neu und unerwartet ist, dass auch membranständige SOD eine dominante und steuernde Funktion bei der Kontrolle der ROS-vermittelten Apoptoseinduktion in Tumorzellen besitzt und dass die Hemmung von SOD durch Antikörper zu einer effizienten und spezifischen ROS-vermittelten Apoptoseinduktion in Tumorzellen führt. Dieser unerwartete Befund erweitert das Bild der Komplexität der Kontrolle des ROS-Signaling. Es wird erkennbar, dass sich SOD und Katalase bei der Kontrolle beider Signalwege sehr effizient und in komplementärer Weise ergänzen (siehe Fig. 2). Es ist allerdings nicht ohne weiteres ersichtlich, wie die jeweils alleinige Hemmung von SOD oder Katalase zu einer wirkungsvollen Apoptoseinduktion führen kann, da ja stets der komplementäre Schutzmechanismus noch wirken sollte. Der Schlüssel zum Verständnis dieses Sachverhaltes wird durch die hier vorgestellten neuen Befunde geliefert, die zeigen, dass sich bei Hemmung eines der beiden protektiven Partner zwangsläufig durch den Konzentrationsanstieg bestimmter Signalkomponenten zur parallelen Hemmung des Komplementärpartners kommt (siehe Figuren 5 und 6) und die Signalwege dann ungebremst ablaufen und zur Apoptose führen können.

Die vorliegende Erfindung offenbart eine auf ROS-Signalwegen begründete Tumortherapie, die aus den bisher publizierten Erkenntnissen nicht abzuleiten war.

Neu und unerwartet ist, dass sich bei der Kombination von Antikörpern gegen SOD und Katalase ein sehr deutlicher Synergieeffekt ergibt, der in einem extrem niedrigen Bedarf an Antikörpern resultiert. Neben der spezifischen Antitumorwirkung führt dies zusätzlich zu einer finanzierbaren Tumortherapie auf Antikörperbasis.

Ziel bei der Entwicklung von neuen Arzneimitteln, Wirkstoffen oder Wirkstoffkombinationen ist, dass diese so spezifisch wie irgend möglich wirken sollen, so dass nur geringste Mengen an Wirkstoff verabreicht werden müssen. Dadurch können nicht nur die Kosten niedrig gehalten werden, sondern es werden auch die Nebenwirkungen gering bleiben. Hierzu trägt die vorliegende Erfindung bei.

## Patentansprüche

1. Pharmazeutische Zusammensetzung, **dadurch gekennzeichnet, dass** sie wenigstens zwei Antikörper oder deren biologisch aktive Fragmente enthält, wobei der eine Antikörper gegen die Katalase gerichtet ist und der andere Antikörper gegen die Superoxiddismutase gerichtet ist, wobei sowohl die Katalase, wie auch die Superoxiddismutase gehemmt wird.

2. Pharmazeutische Zusammensetzung nach Anspruch 1, **dadurch gekennzeichnet, dass** der eine Antikörper oder dessen biologische Fragmente gegen das katalytische Zentrum der Katalase gerichtet sind.

3. Pharmazeutische Zusammensetzung nach Anspruch 1, **dadurch gekennzeichnet, dass** der andere Antikörper gegen das katalytische Zentrum der Superoxiddismutase gerichtet ist.

4. Pharmazeutische Zusammensetzung nach einem der Ansprüche 1-3 zur Verwendung bei der Behandlung einer Tumorerkrankung.

5. Pharmazeutische Zusammensetzung nach Anspruch 4, **dadurch gekennzeichnet, dass** die Tumorerkrankung ausgewählt ist aus folgenden Tumorerkrankungen: Karzinome, Lymphome, Ewing-Sarkome und Neuroblastome

6. Pharmazeutische Zusammensetzung nach Anspruch 5,**dadurch gekennzeichnet, dass** es sich um ein Karzinom des Magens handelt.

## Claims

1. A pharmaceutical composition, **characterized in that** it contains at least two antibodies or the biologically active fragments thereof, wherein the one antibody is directed against the catalase and the other antibody is directed against the superoxide dismutase, wherein the catalase as well as the superoxide dismutase is inhibited.

2. The pharmaceutical composition according to claim 1, **characterized in that** the one antibody or the biological fragments thereof are directed against the catalytic center of the catalase.

3. The pharmaceutical composition according to claim 1, **characterized in that** the other antibody is directed against the catalytic center of the superoxide dismutase.

4. The pharmaceutical composition according to anyone of claims 1 - 3 for the use in the treatment of a tumor disease.

5. The pharmaceutical composition according to claim 4, **characterized in that** the tumor disease is selected from the following tumor diseases: carcinomas, lymphomas, Ewing's sarcomas, and neuroblastomas.

6. The pharmaceutical composition according to claim 5, **characterized in that** it is a gastric carcinoma.

## Revendications

1. Composition pharmaceutique, **caractérisée en ce qu'**elle contient au moins deux anticorps ou leurs fragments biologiquement actifs, le premier anticorps étant dirigé contre la catalase et l'autre anticorps étant dirigé contre la superoxyde dismutase, inhibant aussi bien la catalase que la superoxyde dismutase.

2. Composition pharmaceutique selon la revendication 1, **caractérisée en ce que** le premier anticorps ou ses fragments biologiques sont dirigés contre le centre catalytique de la catalase.

3. Composition pharmaceutique selon la revendication 1, **caractérisée en ce que** l'autre anticorps est dirigé contre le centre catalytique de la superoxyde dismutase.

4. Composition pharmaceutique selon l'une quelconque des revendications 1 à 3 destinée à être utilisée pour traiter une maladie tumorale.

5. Composition pharmaceutique selon la revendication 4, **caractérisée en ce que** la maladie tumorale est choisie parmi les maladies tumorales suivantes comme les carcinomes, les lymphomes, les sarcomes d'Ewing et les neuroblastomes.

6. Composition pharmaceutique selon la revendication 5, **caractérisée en ce qu'**il s'agit d'un cancer gastrique.
